# EUROPEAN PATENT APPLICATION

(11) **EP 4 682 905 A1**
(43) Date of publication of application: **21.01.2026**
(21) Application number: 24771013.0
(22) Date of filing: 15.03.2024
(51) Int. Cl.: G16H 50/50, A61B 10/00, G16H 50/20

(54) **INFORMATION PROCESSING DEVICE, INFORMATION PROCESSING METHOD, PROGRAM, AND STORAGE MEDIUM**

(30) Priority: 16.03.2023 JP 2023042429
(71) Applicant: Eisai R&D Management Co., Ltd., Tokyo 112-8088 (JP)
(72) Inventor: AOSHIMA, Ken, Tsukuba-shi, Ibaraki 300-2635 (JP); NAKAMURA, Yoshitaka, Tokyo 112-8088 (JP); MIURA, Yuji, Tokyo 112-8088 (JP); ANABUKI, Kenichi, Tokyo 112-8088 (JP); DAIRIKI, Ryo, Tokyo 112-8088 (JP); TSUNEYOSHI, Momoka, Tokyo 112-8088 (JP); SEGAWA, Emiko, Tokyo 112-8088 (JP); NAGAOKA, Kazuya, Tokyo 112-8088 (JP)
(74) Representative: Hoffmann Eitle
(86) International application number: PCT/JP2024/010334
(87) International publication number: WO 2024/190911

(57) **Abstract**

An information processing device inputs information of a patient to a prediction model trained using training data, in which at least four variables that fall under at least any of first information relating to orientation, second information relating to memory, third information relating to memory of a holiday, a family gathering, a reservation, or schedule of taking medication in IADL, or memory of a meal or actual work of travel in IADL, and fourth information relating to positive/negative of a biomarker are associated with presence/absence of advance of a symptom of mild cognitive impairment (MCI) or mild Alzheimer's dementia (mild AD), with the information of the patient corresponding to each of the at least four variables, and predicts a probability of advance of a symptom of mild cognitive impairment (MCI) or mild Alzheimer's dementia (mild AD) in the patient. The at least four variables include a first variable that is information relating to orientation relating to time out of the first information, a second variable that is information relating to delayed recall out of the second information, a third variable that is information relating to memory regarding a holiday, a family gathering, a reservation, or schedule for taking medication in IADL, out of the third information, and a fourth variable that is information relating to amyloid β positive/negative out of the fourth information.

## Description

### Technical Field

The present invention relates to an information processing device, an information processing method, a program, and a storage medium.

### Background Art

Conventionally, various types of technology for predicting degree of symptoms of dementia have been proposed. For example, Patent Literature 1 discloses predicting severity of dementia without performing a Mini Mental State Examination (MMSE) used in screening tests for dementia and evaluation of the severity, by analyzing free conversation performed by a patient.

### Citation List

### Patent Literature

Patent Literature 1: Patent Publication JP-A-2020-42659

### Summary of Invention

### Technical Problem

Now, as of recent, there is demand for predicting diseases of mild cognitive impairment (MCI) or mild Alzheimer's dementia (mild AD) patients, who are in line for dementia, in addition to disease prediction for dementia, in order to improve effectiveness of treatment for dementia patients. However, accurately predicting diseases of mild cognitive impairment (MCI) or mild Alzheimer's dementia (mild AD) patients has been considered to be difficult, as the symptoms are lighter in comparison with dementia patients. Also, performing prediction using simple variables is important from the perspective of lightening the processing load on patients and equipment, and so forth, for performing prediction of diseases.

The present invention has been made in light of the foregoing circumstances, and an object thereof is to provide an information processing device, an information processing method, a program, and a storage medium, that can accurately predict probability of advance of symptoms of mild cognitive impairment (MCI) or mild Alzheimer's dementia (mild AD), while lightening the processing load on patients and equipment.

### Solution to Problem

The present disclosure provides an information processing device that inputs information of a patient to a prediction model trained using training data, in which at least four variables that fall under at least any of first information relating to orientation, second information relating to memory, third information relating to memory of a holiday, a family gathering, a reservation, or schedule of taking medication in IADL, or memory of a meal or actual work of travel in IADL, and fourth information relating to positive/negative of a biomarker are associated with presence/absence of advance of a symptom of mild cognitive impairment (MCI) or mild Alzheimer's dementia (mild AD), with the information, which is input, of the patient corresponding to each of the at least four variables, and that predicts a probability of advance of a symptom of mild cognitive impairment (MCI) or mild Alzheimer's dementia (mild AD) in the patient. The at least four variables include a first variable that is information relating to orientation relating to time out of the first information, a second variable that is information relating to delayed recall out of the second information, a third variable that is information relating to memory regarding a holiday, a family gathering, a reservation, or schedule for taking medication in IADL, out of the third information, and a fourth variable that is information relating to amyloid β positive/negative out of the fourth information.

The at least four variables may be four variables which are the first variable, the second variable, the third variable, and the fourth variable.

The at least four variables may be five variables which are the first variable, the second variable, the third variable, the fourth variable, and a fifth variable that is information of any of the first information, the second information, and the third information.

The fifth variable may be information relating to orientation of a place out of the first information.

The fifth variable may be information relating to memory of a name or a type of an object out of the second information.

The fifth variable may be information relating to memory of actual work of meals in IADL out of the third information.

The fifth variable may be information relating to memory of actual work of travel in IADL out of the third information.

The present invention provides an information processing method including the steps of: acquiring patient information; predicting a probability of advance of a symptom of mild cognitive impairment (MCI) or mild Alzheimer's dementia (mild AD) in a patient, by inputting information to a prediction model trained using training data, in which at least four variables that fall under at least any of first information relating to orientation, second information relating to memory, third information relating to memory of a holiday, a family gathering, a reservation, or schedule of taking medication in IADL, or memory of a meal or actual work of travel in IADL, and fourth information relating to positive/negative of a biomarker are associated with presence/absence of advance of a symptom of mild cognitive impairment (MCI) or mild Alzheimer's dementia (mild AD), with the information that is input corresponding to each of the at least four variables included in the patient information; and outputting a prediction result relating to the probability of advance of a symptom of mild cognitive impairment (MCI) or mild Alzheimer's dementia (mild AD) in the patient. The at least four variables include a first variable that is information relating to orientation relating to time out of the first information, a second variable that is information relating to delayed recall out of the second information, a third variable that is information relating to memory regarding a holiday, a family gathering, a reservation, or schedule for taking medication in IADL, out of the third information, and a fourth variable that is information relating to amyloid β positive/negative out of the fourth information.

The present invention provides a program causing a computer to execute: processing of acquiring patient information; processing of predicting a probability of advance of a symptom of mild cognitive impairment (MCI) or mild Alzheimer's dementia (mild AD) in a patient, by inputting information to a prediction model trained using training data, in which at least four variables that fall under at least any of first information relating to orientation, second information relating to memory, third information relating to memory of a holiday, a family gathering, a reservation, or schedule of taking medication in IADL, or memory of a meal or actual work of travel in IADL, and fourth information relating to positive/negative of a biomarker are associated with presence/absence of advance of a symptom of mild cognitive impairment (MCI) or mild Alzheimer's dementia (mild AD), with the information that is input corresponding to each of the at least four variables included in the patient information; and processing of outputting a prediction result relating to the probability of advance of a symptom of mild cognitive impairment (MCI) or mild Alzheimer's dementia (mild AD) in the patient. The at least four variables include a first variable that is information relating to orientation relating to time out of the first information, a second variable that is information relating to delayed recall out of the second information, a third variable that is information relating to memory regarding a holiday, a family gathering, a reservation, or schedule for taking medication in IADL, out of the third information, and a fourth variable that is information relating to amyloid β positive/negative out of the fourth information.

The present invention provides a computer-readable non-transitory storage medium, storing the program of the above configuration.

### Advantageous Effect of Invention

According to the present invention, the probability of advance of symptoms of mild cognitive impairment (MCI) or mild Alzheimer's dementia (mild AD) can be accurately predicted, while lightening the processing load on patients and equipment.

### Brief Description of Drawings

[Figure 1] Figure 1 is a block diagram illustrating an example of a configuration of an information processing device according to the present embodiment.
[Figure 2] Figure 2 is a diagram for describing an example of a learning phase of a prediction model.
[Figure 3] Figure 3 is a diagram illustrating an example of an evaluation phase of the prediction model.
[Figure 4] Figure 4 is a diagram for describing an example of evaluation indices for the prediction model.
[Figure 5] Figure 5 is a diagram illustrating an example of an execution phase of the prediction model.
[Figure 6] Figure 6 is a flowchart illustrating an example of processing for predicting probability of advance of symptoms of mild cognitive impairment (MCI) or mild Alzheimer's dementia (mild AD) in patients.
[Figure 7] Figure 7 is a graph showing an example of temporal change in the degree of advance of symptoms of mild cognitive impairment (MCI) or mild Alzheimer's dementia (mild AD).
[Figure 8] Figure 8 is a diagram for describing an evaluation method by MMSE.
[Figure 9] Figure 9 is a diagram for describing an evaluation method by FAQ.
[Figure 10] Figure 10 is a diagram for describing an evaluation method by ADAS.
[Figure 11] Figure 11 is a diagram for describing contents of IADL.
[Figure 12] Figure 12 is a diagram for describing an example of attribute information of training data.
[Figure 13] Figure 13 is a diagram showing an example of data used for evaluation of a prediction model according to Example 1.
[Figure 14] Figure 14 is a diagram showing an example of data used for evaluation of a prediction model according to Example 1.
[Figure 15] Figure 15 is a diagram showing an example of data used for evaluation of a prediction model according to a comparative example.
[Figure 16] Figure 16 is a diagram showing an example of data used for evaluation of a prediction model according to a comparative example.
[Figure 17] Figure 17 is a diagram showing an example of data used for evaluation of a prediction model according to a comparative example.
[Figure 18] Figure 18 is a diagram showing an example of data used for evaluation of a prediction model according to a comparative example.
[Figure 19] Figure 19 is a diagram showing an example of data used for evaluation of a prediction model according to a comparative example.
[Figure 20] Figure 20 is a diagram showing an example of data used for evaluation of a prediction model according to a comparative example.
[Figure 21] Figure 21 is a diagram showing an example of data used for evaluation of a prediction model according to a comparative example.
[Figure 22] Figure 22 is a diagram showing an example of data used for evaluation of a prediction model according to a comparative example.
[Figure 23] Figure 23 is a diagram showing an example of data used for evaluation of a prediction model according to a comparative example.
[Figure 24] Figure 24 is a diagram showing an example of data used for evaluation of a prediction model according to a comparative example.
[Figure 25] Figure 25 is a diagram showing an example of data used for evaluation of a prediction model according to a comparative example.
[Figure 26] Figure 26 is a diagram showing an example of data used for evaluation of a prediction model according to a comparative example.
[Figure 27] Figure 27 is a diagram showing an example of data used for evaluation of a prediction model according to a comparative example.
[Figure 28] Figure 28 is a diagram showing an example of data used for evaluation of a prediction model according to a comparative example.
[Figure 29] Figure 29 is a diagram showing an example of data used for evaluation of a prediction model according to a comparative example.
[Figure 30] Figure 30 is a diagram showing an example of data used for evaluation of a prediction model according to a comparative example.
[Figure 31] Figure 31 is a diagram showing an example of data used for evaluation of a prediction model according to Example 2.
[Figure 32] Figure 32 is a diagram showing an example of data used for evaluation of a prediction model according to Example 2.
[Figure 33] Figure 33 is a diagram showing an example of data used for evaluation of a prediction model according to a comparative example.
[Figure 34] Figure 34 is a diagram showing an example of data used for evaluation of a prediction model according to a comparative example.
[Figure 35] Figure 35 is a diagram showing an example of data used for evaluation of a prediction model according to a comparative example.
[Figure 36] Figure 36 is a diagram showing an example of data used for evaluation of a prediction model according to a comparative example.
[Figure 37] Figure 37 is a diagram showing an example of data used for evaluation of a prediction model according to a comparative example.
[Figure 38] Figure 38 is a diagram showing an example of data used for evaluation of a prediction model according to a comparative example.
[Figure 39] Figure 39 is a diagram showing an example of data used for evaluation of a prediction model according to a comparative example.
[Figure 40] Figure 40 is a diagram showing an example of data used for evaluation of a prediction model according to a comparative example.
[Figure 41] Figure 41 is a diagram showing an example of data used for evaluation of a prediction model according to a comparative example.
[Figure 42] Figure 42 is a diagram showing an example of data used for evaluation of a prediction model according to a comparative example.
[Figure 43] Figure 43 is a diagram showing an example of data used for evaluation of a prediction model according to a comparative example.
[Figure 44] Figure 44 is a diagram showing an example of data used for evaluation of a prediction model according to a comparative example.
[Figure 45] Figure 45 is a diagram showing an example of data used for evaluation of a prediction model according to a comparative example.
[Figure 46] Figure 46 is a diagram showing an example of data used for evaluation of a prediction model according to a comparative example.
[Figure 47] Figure 47 is a diagram showing an example of data used for evaluation of a prediction model according to a comparative example.
[Figure 48] Figure 48 is a diagram showing an example of data used for evaluation of a prediction model according to a comparative example.
[Figure 49] Figure 49 is a diagram illustrating an example of a hardware configuration of the information processing device according to the present embodiment.

### Description of Embodiments

An embodiment of an information processing device will be described below with reference to the drawings. Note that the present disclosure is not limited to the embodiment described below.

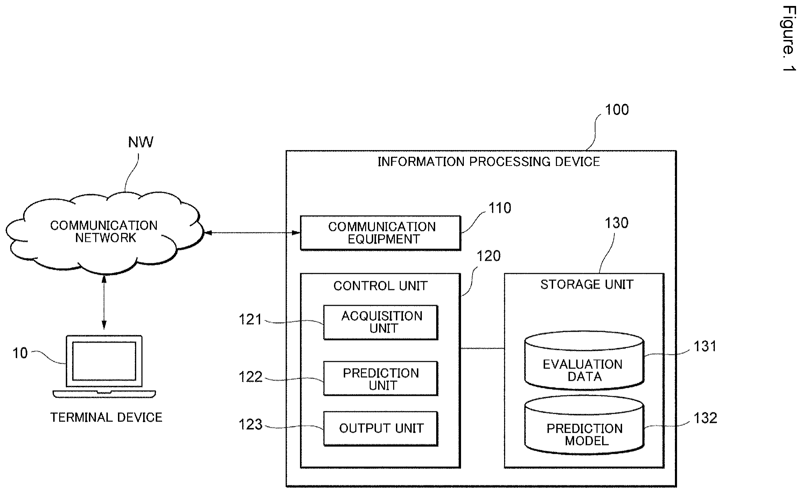

As illustrated in Figure 1, an information processing device 100 is, for example, connected to a terminal device 10 via a communication network NW. The communication network NW is configured of communication equipment, a wireless communication network, and so forth.

The terminal device 10 is a device into which a physician enters patient information, and is configured of a computer terminal device or a mobile information terminal device, for example.

The information processing device 100 includes, for example, communication equipment 110, a control unit 120, and a storage unit 130. The control unit 120 is realized by, for example, a hardware processor such as a CPU (Central Processing Unit) or the like executing a program (software). Also, part or all these configuration elements may be realized by hardware (including circuitry) such as LSI (Large Scale Integration), ASIC (Application Specific Integrated Circuit), FPGA (Field-Programmable Gate Array), GPU (Graphics Processing Unit), or the like, and may be realized by collaboration between the software and the hardware. The program may be stored in the storage unit 130 of the information processing device 100 in advance, or may be stored in a detachably mountable computer-readable recording medium such as a DVD, a CD-ROM, or the like, and be installed in the storage unit 130 of the information processing device 100 by the computer-readable recording medium being mounted to a drive device.

The communication equipment 110 includes a communication interface such as a NIC (Network Interface Card) or the like. The communication equipment 110 communicates with the terminal device 10 using, for example, a cellular network, a Wi-Fi (registered trademark) network, or the like.

The control unit 120 includes, for example, an acquisition unit 121, a prediction unit 122, and an output unit 123.

The acquisition unit 121 acquires patient information transmitted from the terminal device 10 via the communication network NW. Here, the patient information is information that is used for predicting a probability of advance of symptoms of mild cognitive impairment (MCI) or mild Alzheimer's dementia (mild AD) in a patient. Details of the patient information will be described later.

The prediction unit 122 inputs the patient information that is acquired by the acquisition unit 121 into a prediction model 132, and predicts the probability of advance of symptoms of mild cognitive impairment (MCI) or mild Alzheimer's dementia (mild AD) in the patient.

The output unit 123 outputs information relating to the probability of advance of the symptoms of mild cognitive impairment (MCI) or mild Alzheimer's dementia (mild AD) in the patient, which is predicted by the prediction unit 122, to the terminal device 10 via the communication equipment 110. The output unit 123 may output information relating to the probability of advance of the symptoms of mild cognitive impairment (MCI) or mild Alzheimer's dementia (mild AD) in the patient, which is predicted by the prediction unit 122, to a terminal device that a healthcare professional such as a physician or the like or the patient is in possession of, in accordance with a request from the patient, for example.

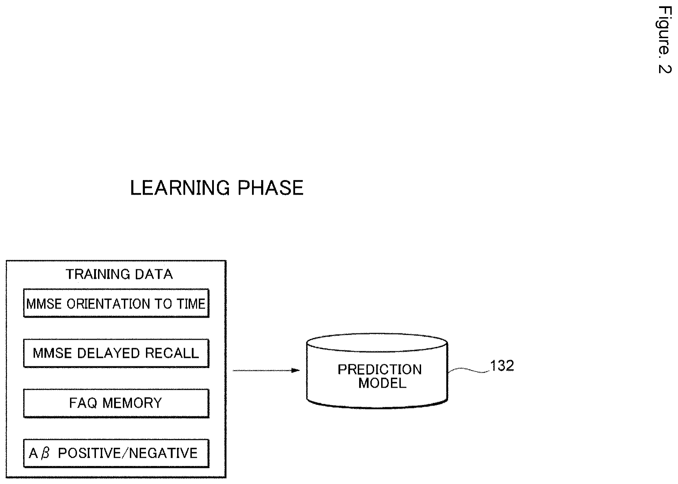

Figure 2 is a diagram illustrating an example of a learning phase of the prediction model 132.

In the example illustrated in Figure 2, out of cohort data (ADNI), which will be described later, relating to each of a plurality of subjects, attribute information of the subjects is taken as a first data group, diagnosis information relating to mild cognitive impairment (MCI) or mild Alzheimer's dementia (mild AD) of the subjects is taken as a second data group, and the prediction model 132 is trained using data extracted with the first data group and the second data group associated. In the present embodiment, as an example of the attribute information of the subjects information relating to orientation to time that is evaluated by MMSE (first information), information relating to delayed recall that is evaluated by MMSE (second information), information relating to memory that is evaluated by FAQ (third information), and information relating to Aβ positive/negative (fourth information) are preferably included, which will be described later.

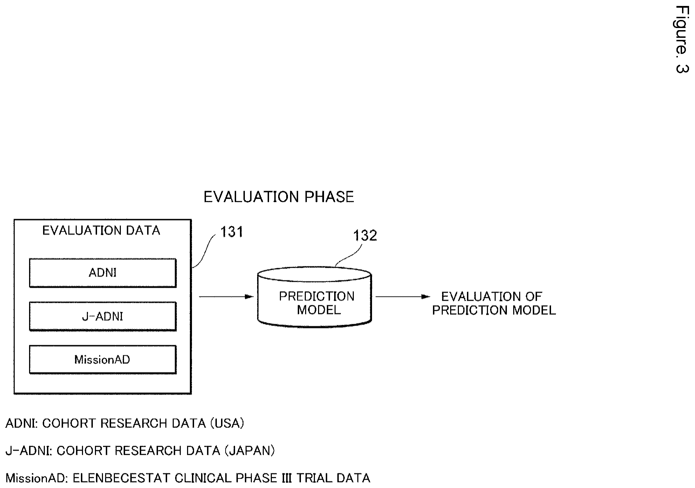

Figure 3 is a diagram illustrating an example of an evaluation phase of the prediction model 132.

In the example illustrated in Figure 3, evaluation of the prediction model 132 that has been trained as described above is performed using evaluation data 131. In the present embodiment, the evaluation data 131 separately uses three databases of ADNI (Alzheimer's Dementia Neuroimaging Initiative), J-ADNI (Japanese Alzheimer's Dementia Neuroimaging Initiative), and MissionAD (Elenbecestat clinical phase III trial data), and the evaluation of the prediction model 132 is individually performed for each database. ADNI is a database based on cohort data collected from a plurality of clinical institutions in order to advance drug trials for dementia-related disease modifying drugs in the USA. J-ADNI is a database based on cohort data collected from a plurality of clinical institutions in order to advance drug trials for dementia-related disease modifying drugs in Japan. MissionAD is a database based on drug trial data that the Present Applicant has independently collected to advance drug trials for dementia-related disease modifying drugs. Each of these databases involve different sets of subjects, and accordingly evaluation of the prediction model 132 using each of these databases is meaningful in judging the scope of patients to which the prediction model 132 is applicable.

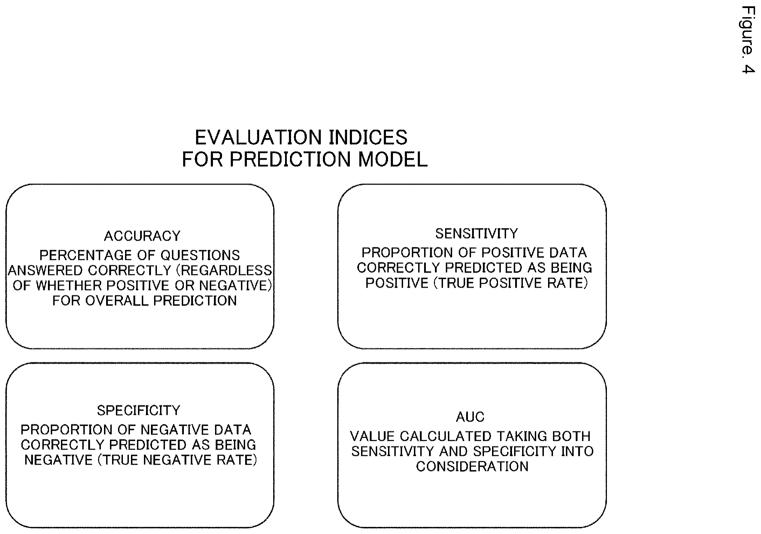

Figure 4 is a diagram for describing an example of evaluation indices for the prediction model 132.

In the example illustrated in Figure 4, accuracy (Accuracy), sensitivity (Sensitivity), specificity (Specificity), and AUC (Area Under the ROC Curve), are listed as examples of evaluation indices of the prediction model 132. Accuracy is an index indicating a percentage of questions answered correctly for the overall prediction, regardless of whether positive or negative. Sensitivity is an index indicating a proportion of positive data that is correctly predicted as being positive (true positive rate). Specificity is an index indicating a proportion of negative data correctly predicted as being negative (true negative rate). AUC is an index that is calculated taking both sensitivity and specificity into consideration. Generally, evaluation based on AUC is preferable for evaluating the overall prediction model 132, and in the present embodiment, a form in which sensitivity is taken into consideration is exemplified, and in another embodiment, a form in which sensitivity and specificity are taken into consideration is exemplified. Further, in yet another embodiment, a form in which accuracy, sensitivity, and specificity are taken into consideration is exemplified. Examples of judgment criteria for acceptable/unacceptable prediction results of the indices include 0.7, 0.67, or 0.6 or higher.

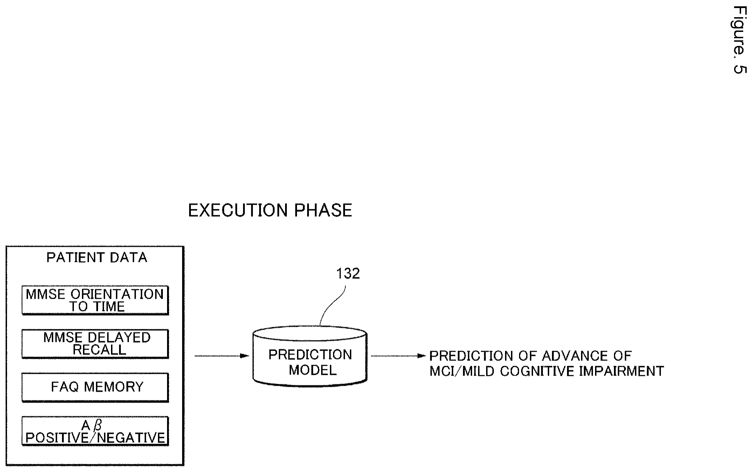

Figure 5 is a diagram illustrating an example of an execution phase of the prediction model 132.

In the example illustrated in Figure 5, patient information that is acquired from the terminal device 10 is input to the prediction model 132 that is trained, as described above, and the probability of the symptoms of mild cognitive impairment (MCI) or mild Alzheimer's dementia (mild AD) advancing in the patient is predicted. In the present embodiment, as an example of attribute information of the patient information, information relating to orientation to time that is evaluated by MMSE (first information), information relating to delayed recall that is evaluated by MMSE (second information), information relating to memory that is evaluated by FAQ (third information), and information relating to Aβ positive/negative (fourth information) are preferably included, which will be described later.

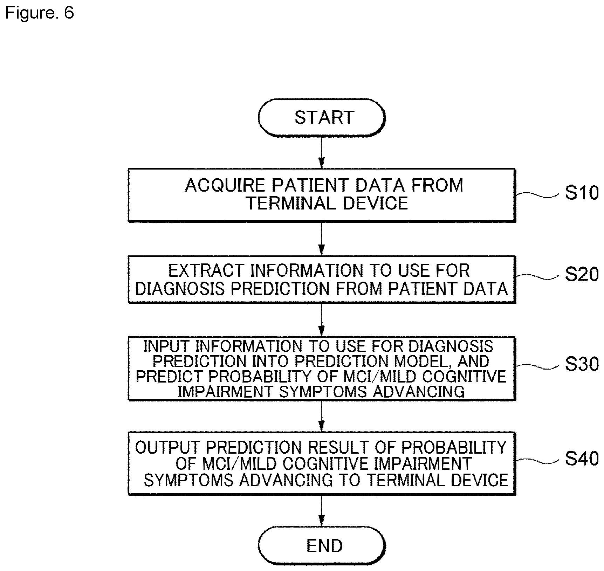

Figure 6 is a flowchart showing an example of processing of predicting a probability of symptoms of mild cognitive impairment (MCI) or mild Alzheimer's dementia (mild AD) advancing in a patient.

As shown in Figure 6, first, the information processing device 100 acquires patient data from the terminal device 10 (step S10).

Next, the information processing device 100 extracts information to be used in diagnosis prediction from the patient data acquired in the previous step S10 (step S20).

Next, the information processing device 100 inputs the information to be used in diagnosis prediction, which was extracted in the previous step S20, into the prediction model 132, to predict the probability of symptoms of mild cognitive impairment (MCI) or mild Alzheimer's dementia (mild AD) advancing in a patient (step S30).

Next, the information processing device 100 outputs prediction results relating to the probability of symptoms of mild cognitive impairment (MCI) or mild Alzheimer's dementia (mild AD) advancing in a patient, from the previous step S30, to the terminal device 10 (step S40), and the flowchart shown in Figure 6 ends.

In the present embodiment, the patient information is information obtained by a physician interviewing a patient, for example, and interviewing techniques include, MMSE (Mini-Mental State Examination), FAQ (Functional activities questionnaire), and ADAS (Alzheimer's Dementia Assessment Scale), for example (see Clinical Practice Guideline for Dementia 2017 (https://www.neurology-jp.org/guidelinem/nintisyo_2017.html), Japanese Journal of Geriatrics 2011; 48:431-438 (https://jpn-geriat-soc.or.jp/publications/other/pdf/review_geriatrics_48_5_431.pdf)).

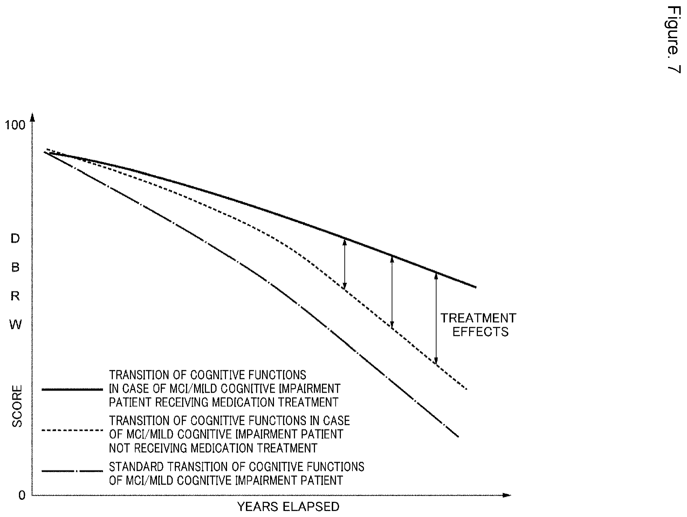

Figure 7 is a graph showing an example of temporal change in degree of advance of symptoms of mild cognitive impairment (MCI) or mild Alzheimer's dementia (mild AD), predicted by the prediction model 132. In the example in this drawing, prediction of onset (prediction of advance of symptoms) of mild cognitive impairment (MCI) or mild Alzheimer's dementia (mild AD) at a point in time in the future is performed over time using the prediction model 132. In this case, prediction models 132 are generated that each perform prediction of onset (prediction of advance of symptoms) of mild cognitive impairment (MCI) or mild Alzheimer's dementia (mild AD), for each point in time in the future, such as for example, half a year later, one year later, two years later, five years later, ten years later, and so forth, on the basis of time-series data of cohort data (ADNI) from the past to the current point in time. Inputting the cohort data (ADNI) at the current point in time to these plurality of prediction models 132 then enables the degree of advance of symptoms of mild cognitive impairment (MCI) or mild Alzheimer's dementia (mild AD) to be consecutively plotted. The graph shown in this example can then be referenced to compare and predict temporal change in the degree of advance of symptoms and effects of treatment by medication, between a case of a patient with mild cognitive impairment (MCI) or mild Alzheimer's dementia (mild AD) receiving treatment by medication and not receiving treatment by medication.

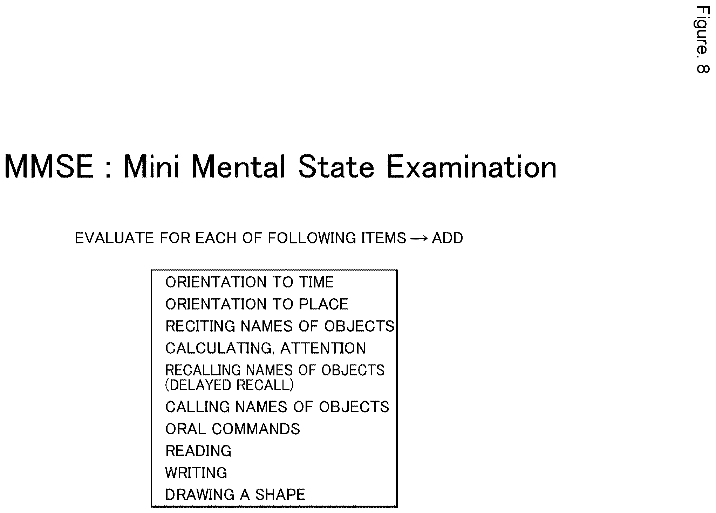

As shown in Figure 8, MMSE is a type of neuropsychological test that is carried out when dementia is suspected. In MMSE, a plurality of diagnostic items are each scored, the scores thereof are added to perform diagnosis of dementia, in which the lower the total score value is, the more advanced this means that the dementia is. Diagnosis items in MMSE include, for example, orientation to time, orientation to place, reciting names of objects, attention, recalling names of objects (delayed recall), calling names of objects, speaking back a sentence, three-stage command, reading, writing, and drawing a shape.

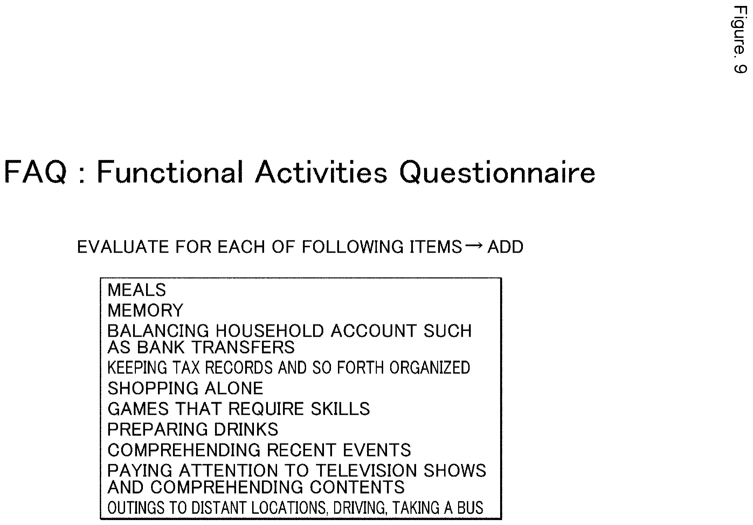

As shown in Figure 9, the FAQ is a type of technique to subjectively evaluate daily living skills of dementia patients. In the FAQ, a plurality of diagnostic items are each scored, the scores thereof are added to perform diagnosis of dementia, in which the higher the total score value is, the lower this means that daily living skills are. The plurality of diagnosis are for evaluating skills regarding, for example, meals, remembering, balancing household account, keeping organized, shopping, games, preparing drinks, comprehending recent events, comprehending contents of television shows, and traveling.

### <Question Items>

- Remembering appointments, family gatherings, holidays, and taking medication (also referred to in the present specification as memory evaluated by FAQ, or simply FAQ memory)
- Preparing a balanced meal (also referred to in the present specification as meals evaluated by FAQ, or simply FAQ meals)
- Going on an outing to a distant location, driving an automobile, taking a bus: travel (also referred to in the present specification as travel evaluated by FAQ, or simply FAQ travel)
- Going shopping alone: shopping (also referred to in the present specification as shopping evaluated by FAQ, or simply FAQ shopping)
- Balancing household accounts, such as bank transfers and so forth
- Organizing tax records and so forth.
- Games that require skill
- Preparing drinks
- Comprehending recent events
- Paying attention to television and so forth, and understanding contents

### <Scoring>

0: Can perform without difficulty
1: Can perform alone, with some difficulty
2: Requires assistance
3: Total assistance

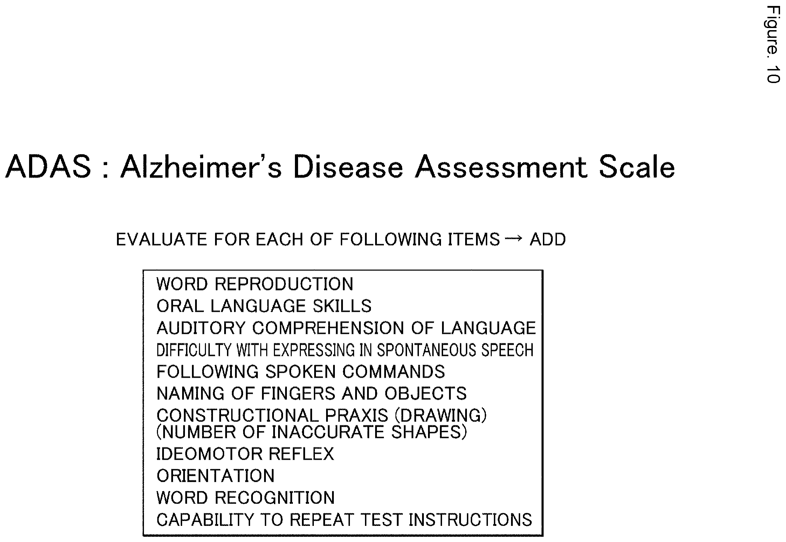

As shown in Figure 10, the ADAS is a test that is consecutively carried out a plurality of times, and change in cognitive functions is evaluated by change in scores. In ADAS, each of a plurality of diagnosis items is scored, and the higher the score thereof is, the more advanced this means that the symptoms are. The plurality of diagnostic items are for evaluating skills related to word reproduction, oral language skills, auditory comprehension of language, difficulty with expressing in spontaneous speech, comprehension of spoken commands, naming of fingers and objects, constructional praxis, ideomotor reflex, orientation, word recognition, and repeating test instructions.

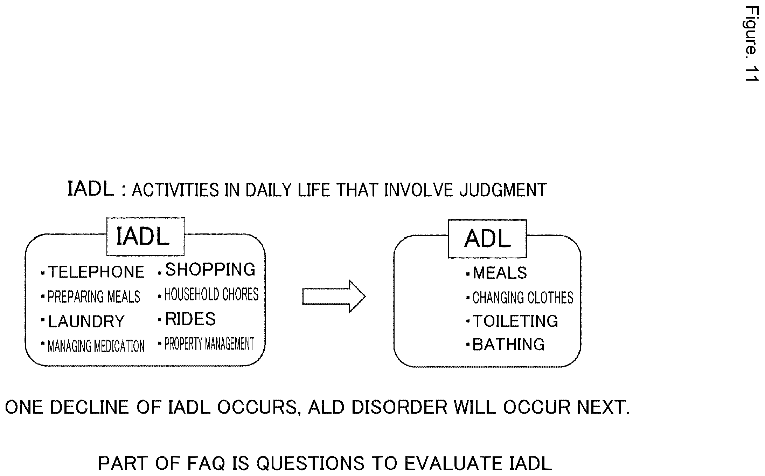

As illustrated in Figure 11, in the present embodiment, the patient information includes, for example, information relating to orientation, information relating to memory, information relating to IADL (Instrumental Activities of Daily Living) (see (https://www.mhlw.go.jp/www1/topics/kenko21_11/s1.html)), and information relating to amyloid β positive/negative.

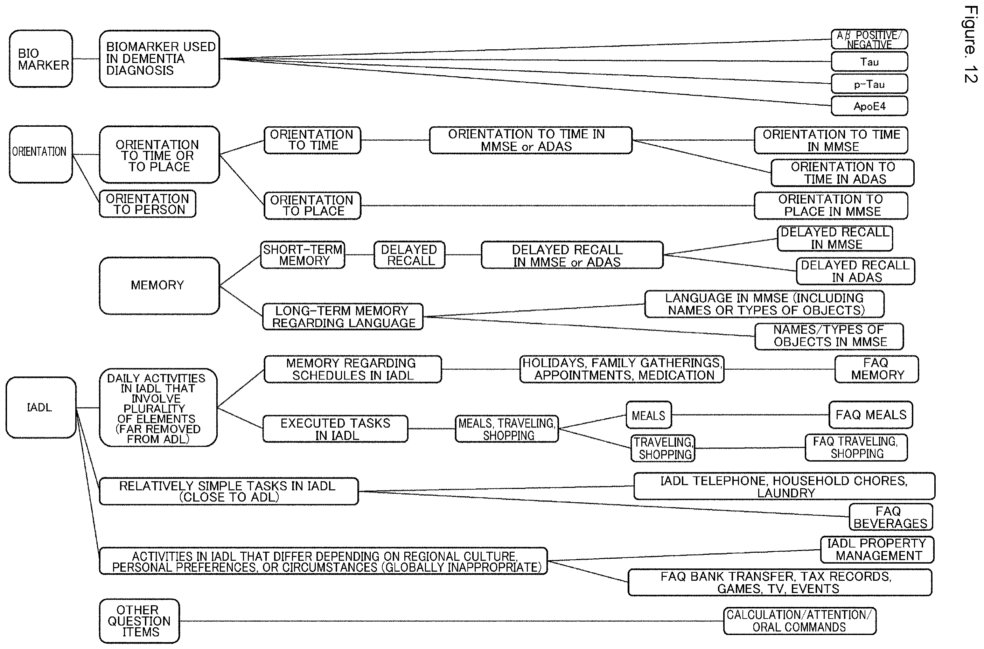

Figure 12 is a diagram for describing an example of attributes information of training data, and indicates branching from higher-order concepts to lower-order concepts, from the left to the right of the page. As shown in Figure 12, information relating to orientation is information relating to skills for comprehensively judging dates and the current time, location and the state of the surroundings, and people, and so forth, to comprehend and understand the situation that he/she is in, and includes, for example, information relating to orientation to time, information relating to orientation to place, and information relating to orientation to person. The information relating to orientation to time is information indicating scores obtained from the patient regarding items relating to orientation to time in the MMSE or the ADAS. The information relating to orientation to place is information indicating scores obtained from the patient regarding items relating to orientation to place in the MMSE, for example.

Information relating to memory is information relating to the capability of the patient to hold short-term or long-term memory, and includes, for example, information related to delayed recall, and information relating to long-term memory regarding language. The information relating to delayed recall is information relating to the capability of remembering things that were learned a predetermined amount of time thereafter. For example, information relating to delayed recall is information indicating scores obtained from the patient, regarding items relating to delayed recall in MMSE and ADAS. The information relating to delayed recall include question items for repeating names of objects. Information relating to long-term memory regarding language is information relating to capability to remember items relating to language over a long period of time, and includes, for example, information relating to the capability of the subject in which the test-giver says three words at one-second intervals each, and the subject repeats these same words (reciting names of objects), information relating to the capability of viewing objects that the test-giver has prepared and accurately saying the names of the things that are viewed (calling names of objects), and information relating to the capability of accurate memory of contents communicated by the test-giver (delayed recall). Information relating to long-term memory regarding language is information indicating scores obtained from the patient regarding items relating to calling names of objects or the like in the MMSE, for example.

Information relating to IADL is information relating to activities in daily life that involve judgment capabilities, and is information for numerical evaluation of capabilities relating to, for example, telephone calls, preparing meals, laundry, management of medications, shopping, household chores, rides, and property management. Generally, decline in evaluation values relating to ADL (Activities of Daily Living: daily life activities) that is an index of dementia, such as, for example, eating, changing clothes, toileting, bathing, and so forth, follow decline in evaluation values relating to IADL, and accordingly information relating to IADL is meaningful information in comprehending early signs of dementia. Information relating to IADL is classified in accordance with whether a daily life activity including a plurality of elements, whether a relatively simple daily life activity, and whether an activity that differs depending on regional culture, or personal preferences or circumstances. Daily life activities including a plurality of elements are activities that are far removed from ADL, and are classified into activities relating to capabilities relating to memory relating to plans in IADL, such as for example, holidays, family gatherings, reservations, management of medications, shopping, and so forth, and activities relating to executed tasks in IADL, such as preparing meals, traveling, and so forth. Information relating to memory relating to plans in IADL is information indicating scores obtained from replies from the patient regarding items of holidays, family gatherings, reservations, management of medications, and so forth, in FAQ, for example. Information relating to executed tasks in IADL is information indicating scores obtained from the patient regarding items such as preparing meals, traveling, and so forth in FAQ, for example. Relatively simple daily life activities are activities close to ADL, and are activities relating to capabilities relating to telephone calls, household chores, laundry, and household chores. Information relating to relatively simple daily life activities in IADL is information indicating scores obtained from the patient regarding, for example, telephone calls, household chores, laundry, and so forth. Also, activities that differ depending on regional culture, or personal preferences or circumstances correspond to capabilities relating to, for example, managing property, games that require skill, and so forth. Information relating to activities that differ depending on regional culture, or personal preferences or circumstances in IADL is information indicating scores obtained from the patient regarding managing property, games that require skill, and so forth, by neuropsychological tests (including FAQ and so forth), for example.

A biomarker is an item or a substance in the living body that serves as an index of whether or not there is a certain disorder, change in pathological conditions, or effects of treatment. Data that is used as biomarkers is primarily biogenetically-derived such as blood pressure, pulse, electrocardiogram, substances such as proteins or the like measured in the blood, and so forth. Information relating to positive/negative of biomarkers may include information relating to evaluation and determination obtained from amyloid β, and may also be information relating to evaluation and determination obtained from Tau, p-Tau, ApoE4, or the like. Tau is a protein that causes dementia through a variety of neurodegenerative disorders such as Alzheimer's dementia and others, by accumulating in the brain and leading to neuronal necrosis. p-Tau means phosphorylated tau protein, and is an abnormal structure that appears in the brains of Alzheimer's dementia patients, with the amount of accumulation thereof correlating with the severity of dementia. ApoE4 is a genetical subtype ε4 (ApoE-ε4) of apolipoprotein E (ApoE), and is gathering attention as a genetic factor that is highly correlated with AD. Information relating to positive/negative regarding biomarkers is information indicating whether or not a biomarker that is a factor highly correlated with onset of dementia has accumulated to a predetermined level in the brain of the patient, and is information that is obtained through, for example, diagnostic imaging or hemodiagnosis (including CSF tests and so forth). In a case in which a biomarker is positive, this suggests that the progression of dementia is advanced as compared to a case in which the biomarker is negative.

Next, examples of the prediction model 132 according to the present embodiment will be described with reference to the drawings. Examples of evaluation indices of the prediction model 132 include accuracy (Accuracy), sensitivity (Sensitivity), specificity (Specificity), and AUC (Area Under the ROC Curve), as described earlier. While the usage method of evaluation indices of the prediction model 132 is not limited in particular, hereinafter, as one example, a case of evaluating the prediction model 132 using the two evaluation indices of sensitivity and AUC is described as an example in a case of using evaluation data of one of ADNI, J-ADNI, and MissionAD, out of these evaluation indices. In the present embodiment, a numerical value of "0.7", which is a generally-used threshold value, will be applied in evaluation of the prediction model 132 using ADNI, J-ADNI, and MissionAD, with prediction models 132 that satisfy both conditions of "sensitivity ≥ 0.7" and "AUC ≥ 0.7" as examples, and prediction models 132 in which at least one condition of "sensitivity ≥ 0.7" and "AUC ≥ 0.7" is not satisfied as comparative examples. It should be noted here that the examples of the present disclosure described below are only one form, and that the examples described below are not limiting.

### (Implementation Conditions)

In construction and evaluation of prediction models, first, a model is constructed in which, out of ADNI data in which various types of information and presence/absence of advance of symptoms are associated, 80% of data is used as training data, and presence/absence of advance of symptoms is predicted. Further, the remaining 20% of data of the ADNI data, and J-ADNI and MissionAD data are taken as evaluation data, and presence/absence of advance of symptoms based on the prediction model and actual presence/absence of advance of symptoms are matched, and various types of evaluation indices are calculated.

### (Example 1)

Data used for evaluation of the prediction model 132 according to Example 1 is data not used for constructing the prediction model 132 out of the training data, and includes at least four variables of falling under first information relating to orientation, second information relating to memory, third information relating to memory of holidays, family gatherings, reservations, or taking medication in IADL, or memory of meals or actual work of travel in IADL, and fourth information relating to biomarker positive/negative. In other words, each variable of the at least four variables falls under at least one of the first information through the fourth information. The at least four variables include a first variable that is information relating to orientation relating to time out of the first information, a second variable that is information relating to delayed recall out of the second information, a third variable that is information relating to memory regarding holidays, family gatherings, reservations, or schedule for taking medication in IADL, out of the third information, and a fourth variable that is information relating to amyloid β positive/negative out of the fourth information.

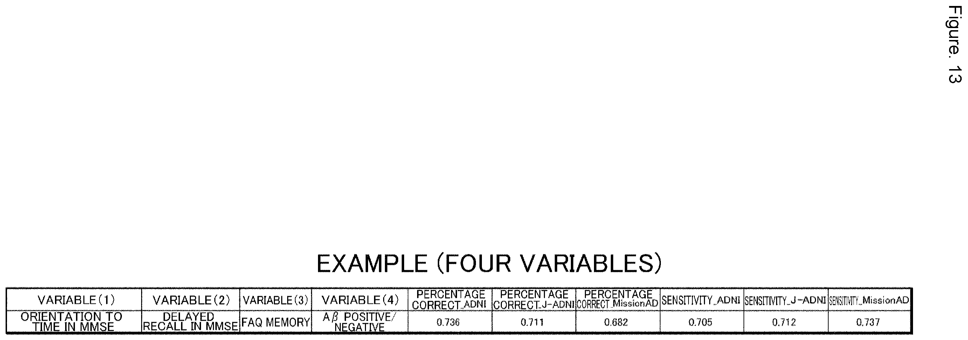

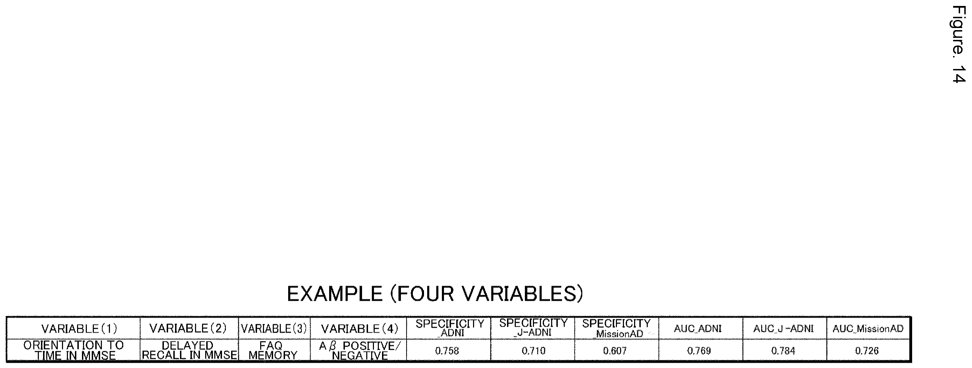

Figure 13 and Figure 14 are diagrams showing an example of data used for evaluation of the prediction model 132 according to Example 1. In the example in Figure 13 and Figure 14, data used for evaluation of the prediction model 132 includes four variables, which are variable (1), variable (2), variable (3), and variable (4). Variable (1) is information relating to orientation to time that is evaluated by MMSE, variable (2) is information relating to delayed recall that is evaluated by MMSE, variable (3) is information relating to memory evaluated by FAQ, and variable (4) is information relating to Aβ positive/negative. In this example, evaluation values of both of sensitivity and AUC are "0.7" or higher in each evaluation of the prediction model 132 using ADNI, J-ADNI, and MissionAD.

### (Comparative Examples)

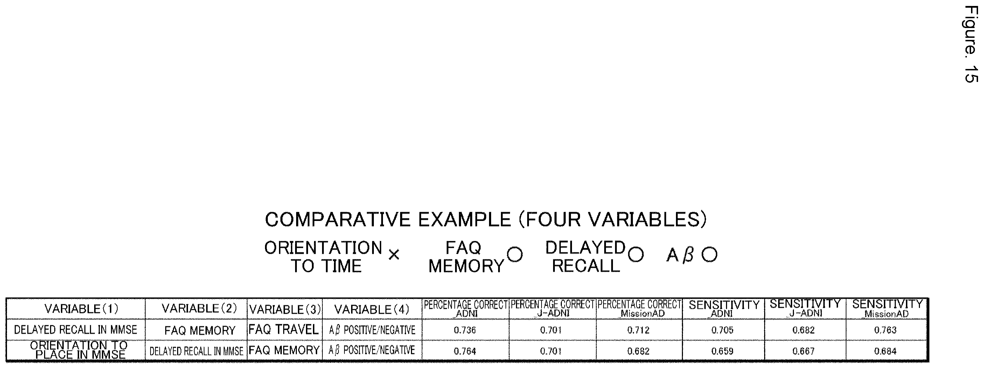

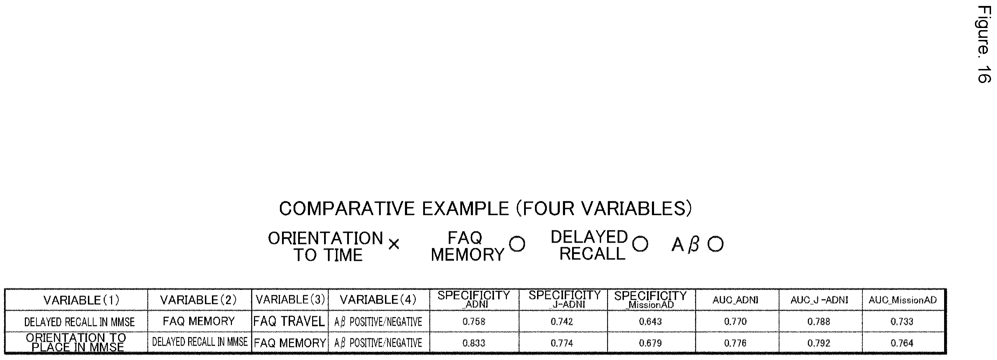

Figure 15 and Figure 16 are diagrams showing an example of data used for evaluation of the prediction model 132 according to a comparative example. In the example in Figure 15 and Figure 16, data used for evaluation of the prediction model 132 includes four variables, which are variable (1), variable (2), variable (3), and variable (4). Variables (1) to (4) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to orientation to time that is evaluated by MMSE out of the four essential types of information making up the training data for the prediction model 132 according to Example 1 shown in Figure 13 and Figure 14, and include information relating to delayed recall that is evaluated by MMSE, information relating to memory evaluated by FAQ, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

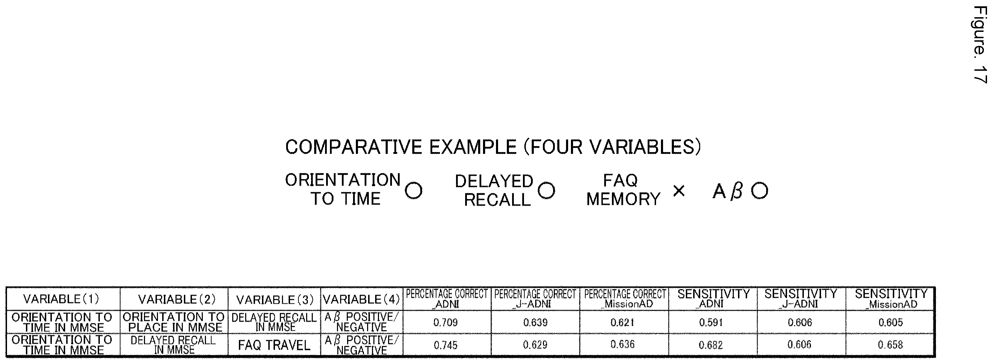

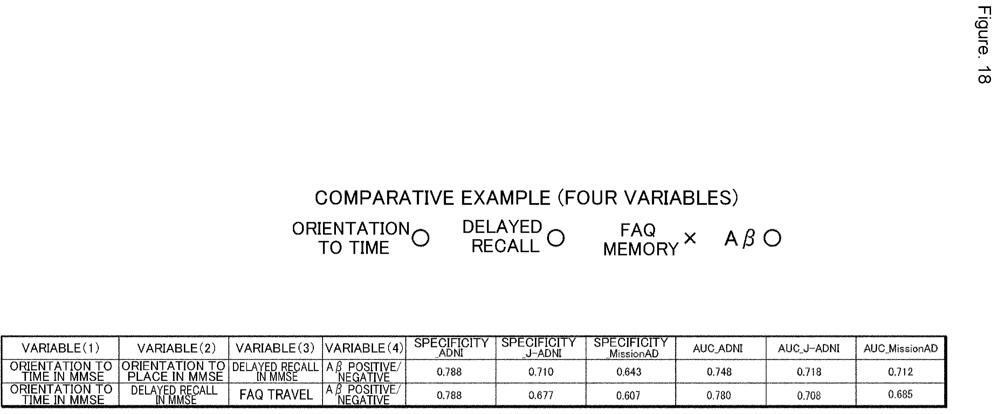

Figure 17 and Figure 18 are diagrams showing an example of data used for evaluation of the prediction model 132 according to a comparative example. In the example in Figure 17 and Figure 18, data used for evaluation of the prediction model 132 includes four variables, which are variable (1), variable (2), variable (3), and variable (4). Variables (1) to (4) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to memory evaluated by FAQ, out of the four essential types of information making up the training data for the prediction model 132 according to Example 1 shown in Figure 13 and Figure 14, and include information relating to orientation to time that is evaluated by MMSE, information relating to delayed recall that is evaluated by MMSE, and information relating to Aβ positive/negative, which are the three remaining types of essential information. In this example, evaluation values of at least one of sensitivity and AUC are lower than "0.7" in each evaluation of prediction model 132 using ADNI, J-ADNI, and MissionAD.

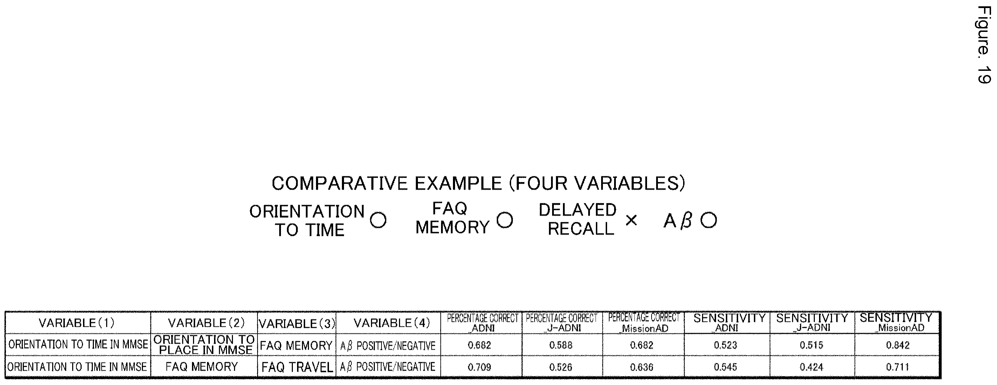

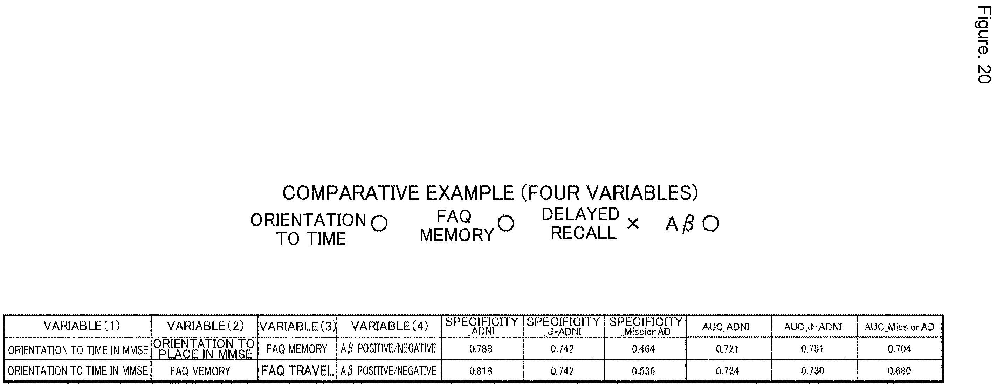

Figure 19 and Figure 20 are diagrams showing an example of data used for evaluation of the prediction model 132 according to a comparative example. In the example in Figure 19 and Figure 20, data used for evaluation of the prediction model 132 includes four variables, which are variable (1), variable (2), variable (3), and variable (4). Variables (1) to (4) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to delayed recall evaluated by MMSE, out of the four essential types of information making up the training data for the prediction model 132 according to Example 1 shown in Figure 13 and Figure 14, and include information relating to orientation to time that is evaluated by MMSE, information relating to memory that is evaluated by FAQ, and information relating to Aβ positive/negative, which are the three remaining types of essential information. In this example, evaluation values of at least one of sensitivity and AUC are lower than "0.7" in each evaluation of prediction model 132 using ADNI, J-ADNI, and MissionAD.

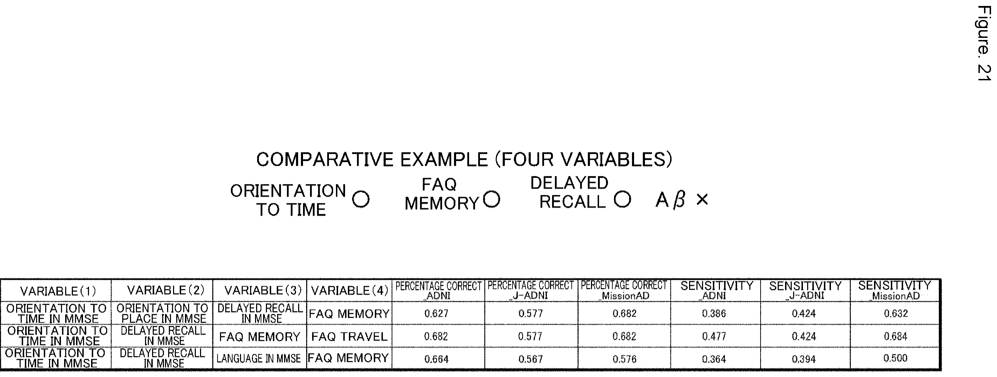

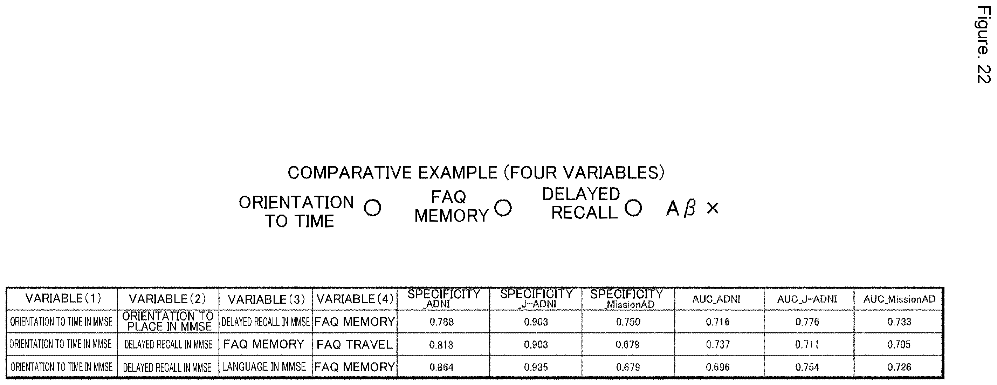

Figure 21 and Figure 22 are diagrams showing an example of data used for evaluation of the prediction model 132 according to a comparative example. In the example in Figure 21 and Figure 22, data used for evaluation of the prediction model 132 includes four variables, which are variable (1), variable (2), variable (3), and variable (4). Variables (1) to (4) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to Aβ positive/negative, out of the four essential types of information making up the training data for the prediction model 132 according to Example 1 shown in Figure 13 and Figure 14, and include information relating to orientation to time that is evaluated by MMSE, information relating to delayed recall evaluated by MMSE, and information relating to memory that is evaluated by FAQ, which are the three remaining types of essential information. In this example, evaluation values of at least one of sensitivity and AUC are lower than "0.7" in each evaluation of the prediction model 132 using ADNI, J-ADNI, and MissionAD.

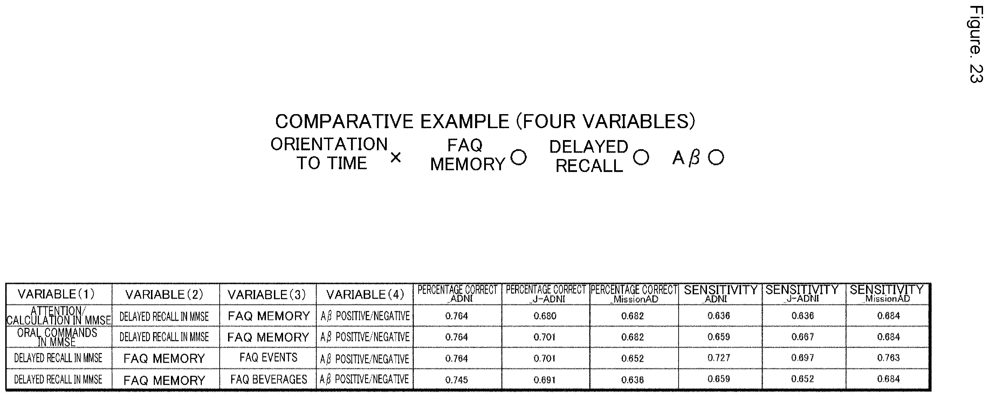

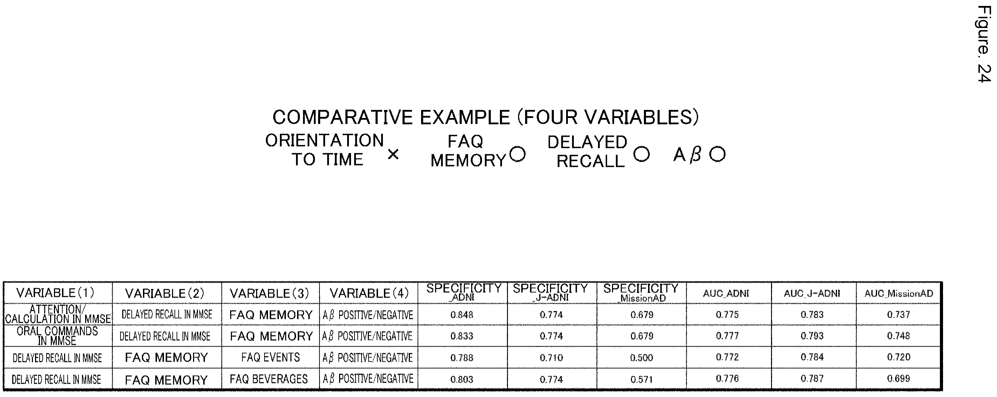

Figure 23 and Figure 24 are diagrams showing an example of data used for evaluation of the prediction model 132 according to a comparative example. In the example in Figure 23 and Figure 24, data used for evaluation of the prediction model 132 includes four variables, which are variable (1), variable (2), variable (3), and variable (4). Variables (1) to (4) include information that does not fall under any of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to orientation to time that is evaluated by MMSE out of the four essential types of information making up the training data for the prediction model 132 according to Example 1 shown in Figure 13 and Figure 14, and include information relating to delayed recall that is evaluated by MMSE, information relating to memory evaluated by FAQ, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

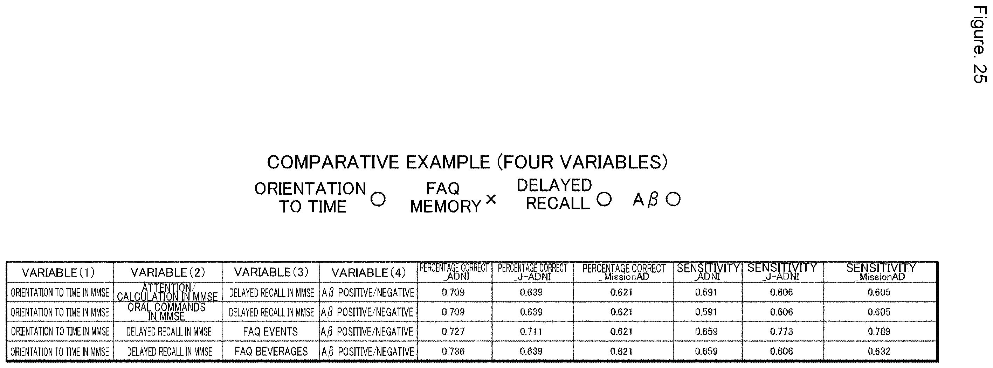

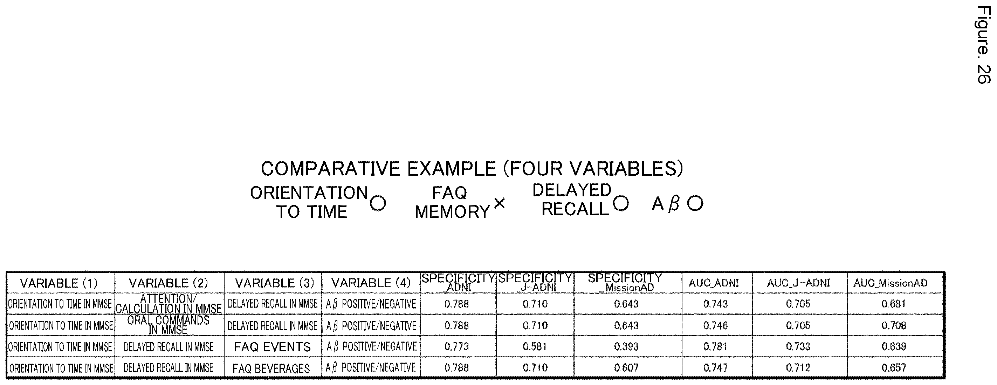

Figure 25 and Figure 26 are diagrams showing an example of data used for evaluation of the prediction model 132 according to a comparative example. In the example in Figure 25 and Figure 26, data used for evaluation of the prediction model 132 includes four variables, which are variable (1), variable (2), variable (3), and variable (4). Variables (1) to (4) include information that does not fall under any of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to memory evaluated by FAQ, out of the four essential types of information making up the training data for the prediction model 132 according to Example 1 shown in Figure 13 and Figure 14, and include information relating to orientation to time that is evaluated by MMSE, information relating to delayed recall that is evaluated by MMSE, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

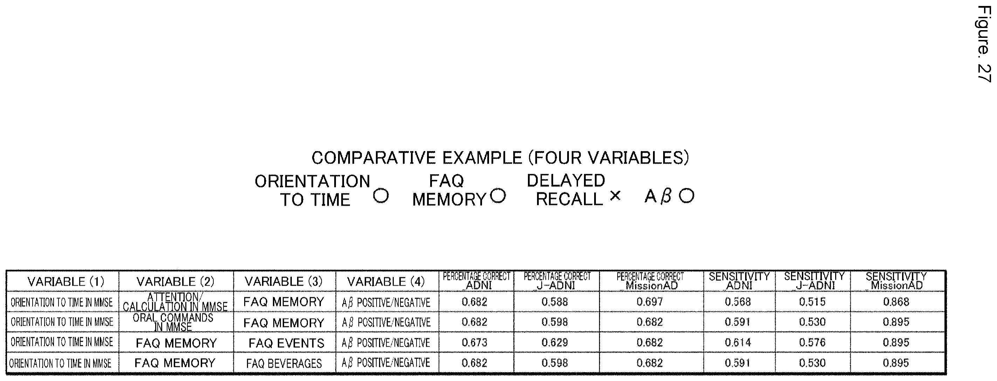

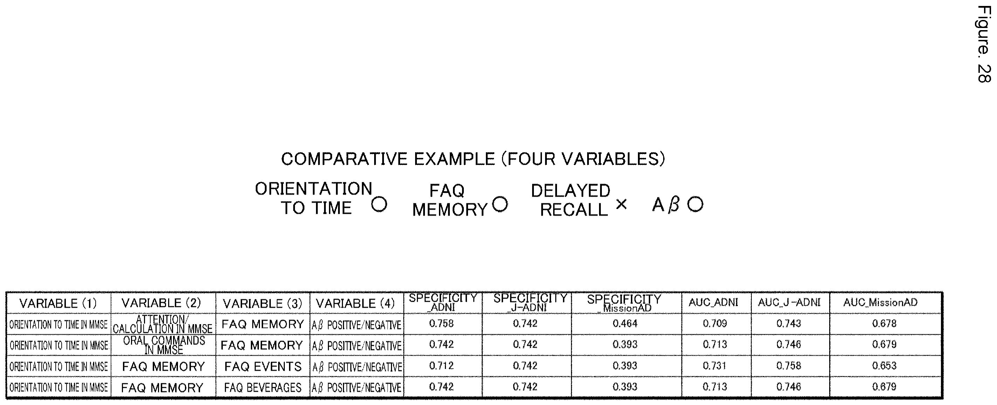

Figure 27 and Figure 28 are diagrams showing an example of data used for evaluation of the prediction model 132 according to a comparative example. In the example in Figure 27 and Figure 28, data used for evaluation of the prediction model 132 includes four variables, which are variable (1), variable (2), variable (3), and variable (4). Variables (1) to (4) include information that does not fall under any one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to delayed recall evaluated by MMSE, out of the four essential types of information making up the training data for the prediction model 132 according to Example 1 shown in Figure 13 and Figure 14, and include information relating to orientation to time that is evaluated by MMSE, information relating to memory that is evaluated by FAQ, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

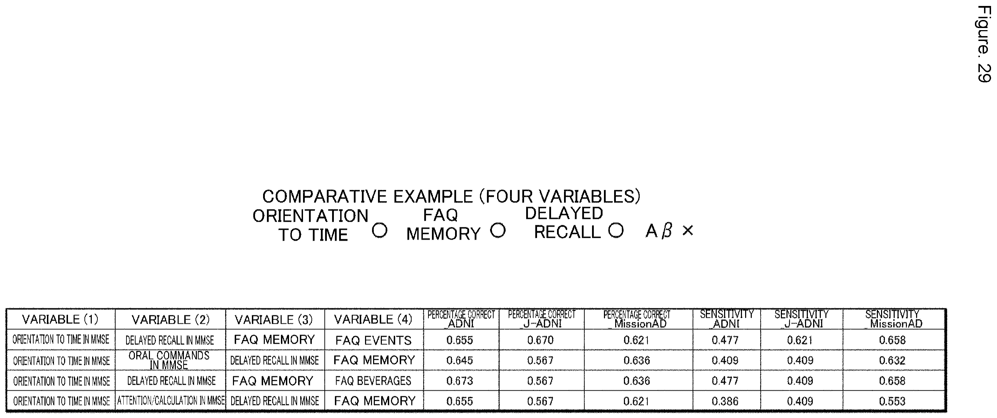

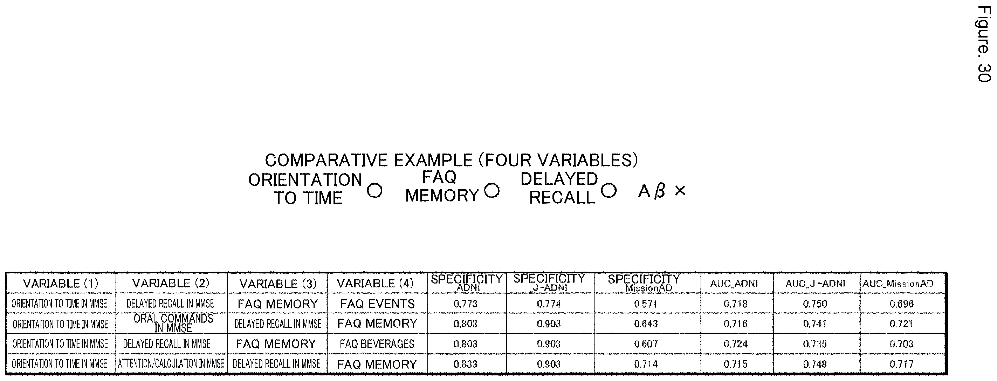

Figure 29 and Figure 30 are diagrams showing an example of data used for evaluation of the prediction model 132 according to a comparative example. In the example in Figure 29 and Figure 30, data used for evaluation of the prediction model 132 includes four variables, which are variable (1), variable (2), variable (3), and variable (4). Variables (1) to (4) include information that does not fall under any one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to Aβ positive/negative, out of the four essential types of information making up the training data for the prediction model 132 according to Example 1 shown in Figure 13 and Figure 14, and include information relating to orientation to time that is evaluated by MMSE, information relating to delayed recall evaluated by MMSE, and information relating to memory that is evaluated by FAQ, which are the three remaining types of essential information. In this example, evaluation values of at least one of sensitivity and AUC are lower than "0.7" in each evaluation of the prediction model 132 using ADNI, J-ADNI, and MissionAD.

### (Example 2)

Data used for evaluation of the prediction model 132 according to Example 2 includes at least four variables of falling under one of first information relating to orientation, second information relating to memory, third information relating to memory of holidays, family gatherings, reservations, or schedule for taking medication in IADL, or memory of meals or actual work of travel in IADL, and fourth information relating to biomarker positive/negative. In other words, each variable of the at least four variables falls under at least one of the first information through the fourth information. The at least four variables are five variables made up of a first variable that is information relating to orientation relating to time out of the first information, a second variable that is information relating to delayed recall out of the second information, a third variable that is information relating to memory regarding holidays, family gatherings, reservations, or schedule for taking medication in IADL, out of the third information, a fourth variable that is information relating to amyloid β positive/negative out of the fourth information, and a fifth variable that is one information of the first information, the second information, and the third information. The fifth variable may be, for example, information relating to orientation to place out of the first information, may be information relating to memory of names or types of objects out of the second information, may be information relating to memory of actual work of meals in IADL out of the third information, and may be information relating to memory of actual work of travel in IADL out of the third information. While an example of using five variables will be described in Example 2 below, yet another variable falling under one information of the first information to the fourth information may be added.

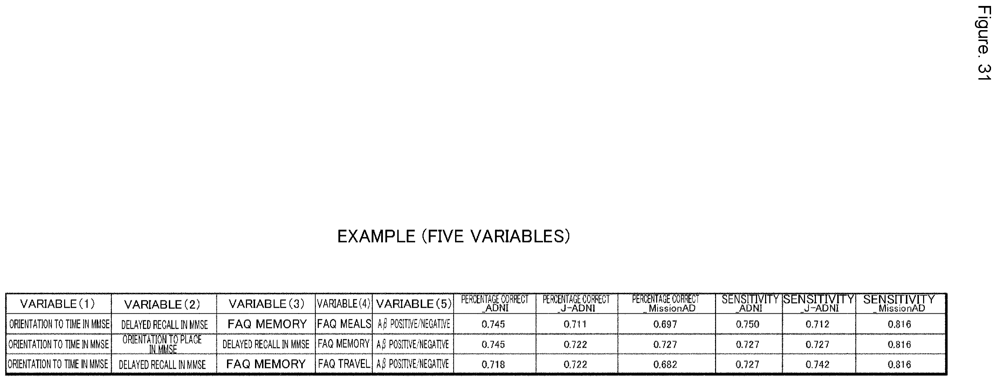

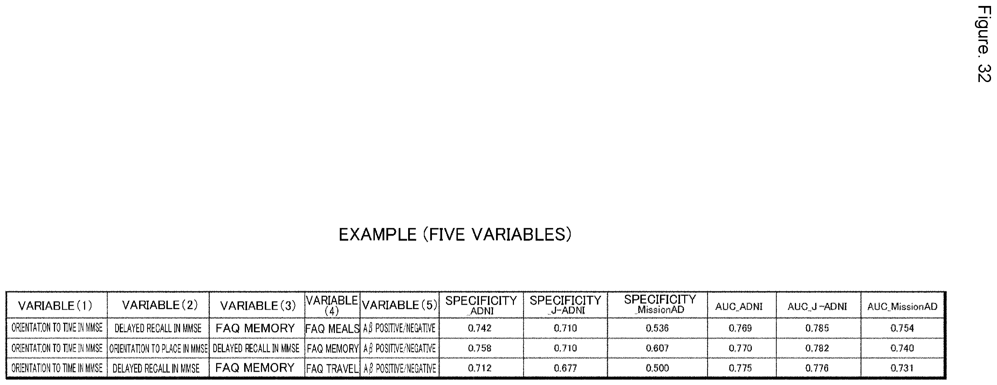

Figure 31 and Figure 32 are diagrams showing an example of data used for evaluation of the prediction model 132 according to Example 2. In the example in Figure 31 and Figure 32, data used for evaluation of the prediction model 132 includes five variables, which are variable (1), variable (2), variable (3), variable (4), and variable (5). In a first dataset, variable (1) is information relating to orientation to time that is evaluated by MMSE, variable (2) is information relating to delayed recall that is evaluated by MMSE, variable (3) is information relating to memory that is evaluated by FAQ, variable (4) is information relating to meals that are evaluated by FAQ, and variable (5) is information relating to Aβ positive/negative. In a second dataset, variable (1) is information relating to orientation to time that is evaluated by MMSE, variable (2) is information relating to orientation to place that is evaluated by MMSE, variable (3) is information relating to delayed recall that is evaluated by MMSE, variable (4) is information relating to memory that is evaluated by FAQ, and variable (5) is information relating to Aβ positive/negative. In a third dataset, variable (1) is information relating to orientation to time that is evaluated by MMSE, variable (2) is information relating to delayed recall that is evaluated by MMSE, variable (3) is information relating to memory evaluated by FAQ, variable (4) is information relating to travel that is evaluated by FAQ, and variable (5) is information relating to Aβ positive/negative. In a fourth dataset, variable (1) is information relating to orientation to time that is evaluated by MMSE, variable (2) is information relating to delayed recall that is evaluated by MMSE, variable (3) is information relating to reciting names of objects that is evaluated by MMSE, variable (4) is information relating to memory that is evaluated by FAQ, and variable (5) is information relating to Aβ positive/negative. In this example, evaluation values of both of sensitivity and AUC are "0.7" or higher in each evaluation of the prediction model 132 using ADNI, J-ADNI, and MissionAD with respect to the first through fourth datasets.

### (Comparative Examples)

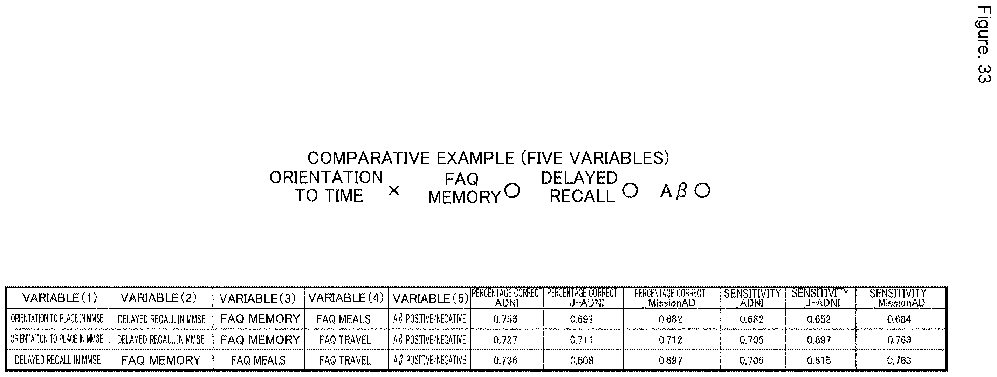

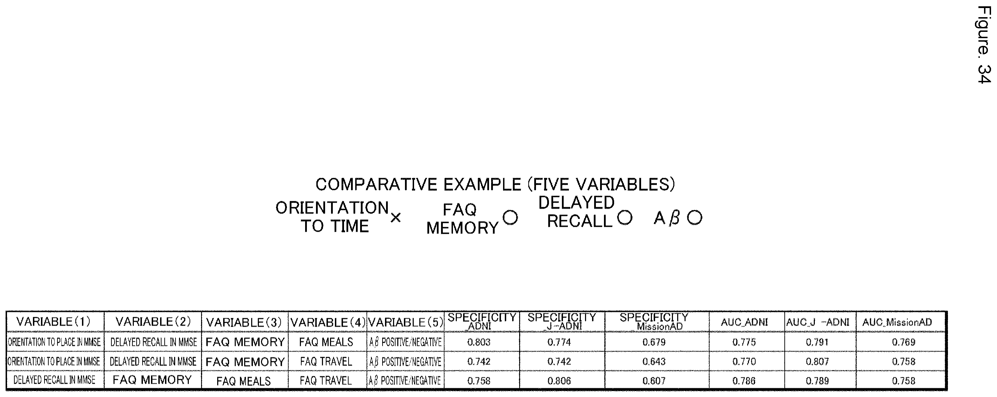

Figure 33 and Figure 34 are diagrams showing an example of data used for evaluation of the prediction model 132 according to a comparative example. In the example in Figure 33 and Figure 34, data used for evaluation of the prediction model 132 includes five variables, which are variable (1), variable (2), variable (3), variable (4), and variable (5). Variables (1) to (5) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (5) do not include information relating to orientation to time that is evaluated by MMSE out of the four essential types of information making up the training data for the prediction model 132 according to Example 2 shown in Figure 31 and Figure 32, and include information relating to delayed recall that is evaluated by MMSE, information relating to memory that is evaluated by FAQ, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

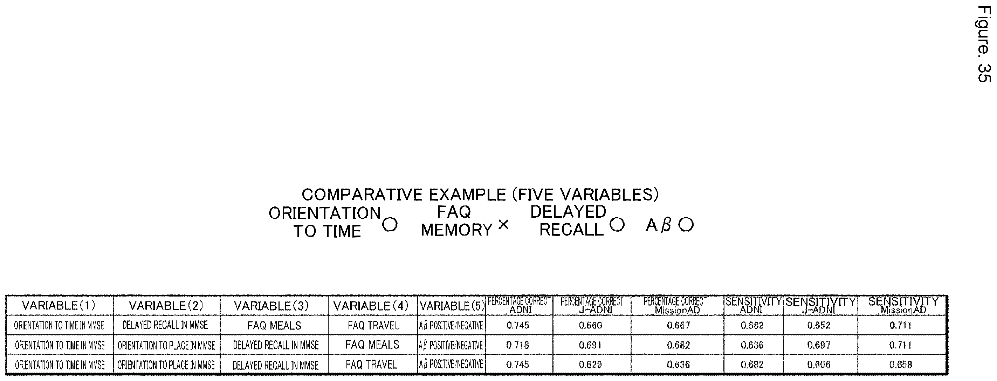

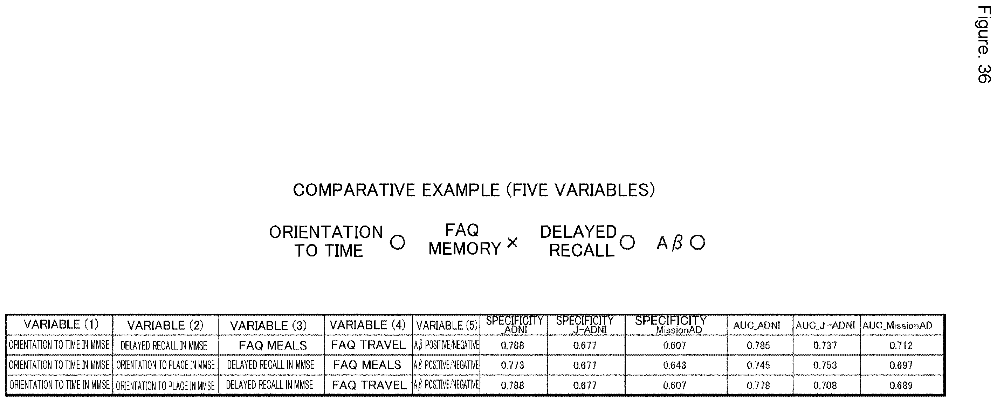

Figure 35 and Figure 36 are diagrams showing an example of data used for evaluation of the prediction model 132 according to a comparative example. In the example in Figure 35 and Figure 36, data used for evaluation of the prediction model 132 includes five variables, which are variable (1), variable (2), variable (3), variable (4), and variable (5). Variables (1) to (5) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (5) do not include information relating to memory that is evaluated by FAQ out of the four essential types of information making up the training data for the prediction model 132 according to Example 2 shown in Figure 31 and Figure 32, and include information relating to orientation to time that is evaluated by MMSE, information relating to delayed recall that is evaluated by MMSE, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

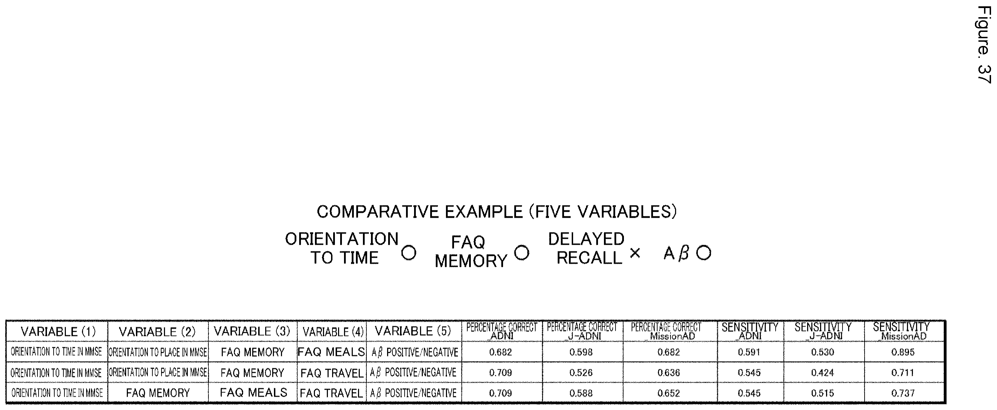

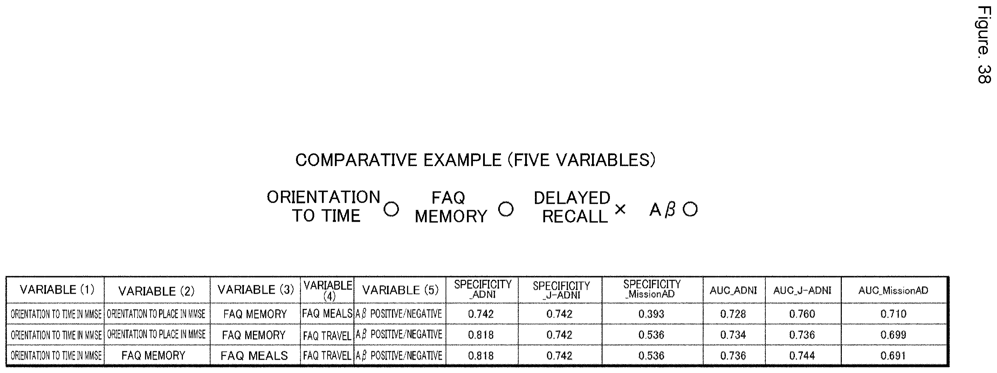

Figure 37 and Figure 38 are diagrams showing an example of data used for evaluation of the prediction model 132 according to a comparative example. In the example in Figure 37 and Figure 38, data used for evaluation of the prediction model 132 includes five variables, which are variable (1), variable (2), variable (3), variable (4), and variable (5). Variables (1) to (5) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (4) do not include information relating to delayed recall that is evaluated by MMSE out of the four essential types of information making up the training data for the prediction model 132 according to Example 2 shown in Figure 31 and Figure 32, and include information relating to orientation to time that is evaluated by MMSE, information relating to memory that is evaluated by FAQ, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

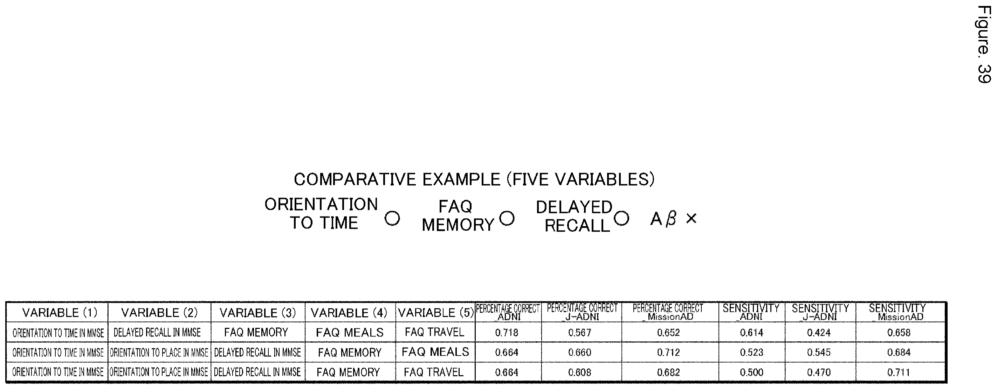

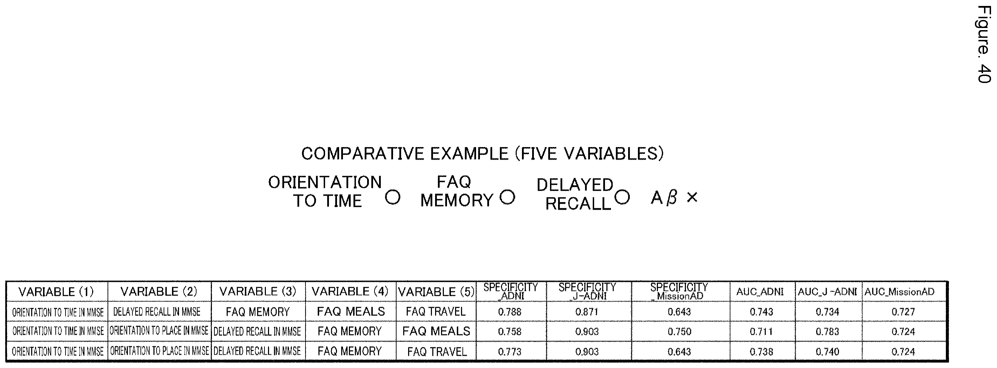

Figure 39 and Figure 40 are diagrams showing an example of data used for evaluation of the prediction model 132 according to a comparative example. In the example in Figure 39 and Figure 40, data used for evaluation of the prediction model 132 includes five variables, which are variable (1), variable (2), variable (3), variable (4), and variable (5). Variables (1) to (5) fall under one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (5) do not include information relating to Aβ positive/negative out of the four essential types of information making up the training data for the prediction model 132 according to Example 2 shown in Figure 31 and Figure 32, and include information relating to orientation to time that is evaluated by MMSE, information relating to delayed recall that is evaluated by MMSE, and information relating to memory that is evaluated by FAQ, which are the three remaining types of essential information. In this example, evaluation values of at least one of sensitivity and AUC are lower than "0.7" in each evaluation of the prediction model 132 using ADNI, J-ADNI, and MissionAD.

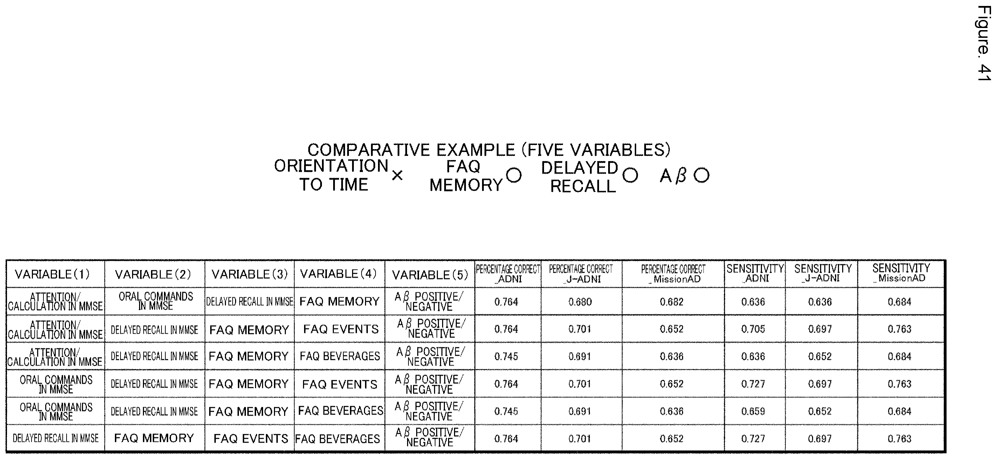

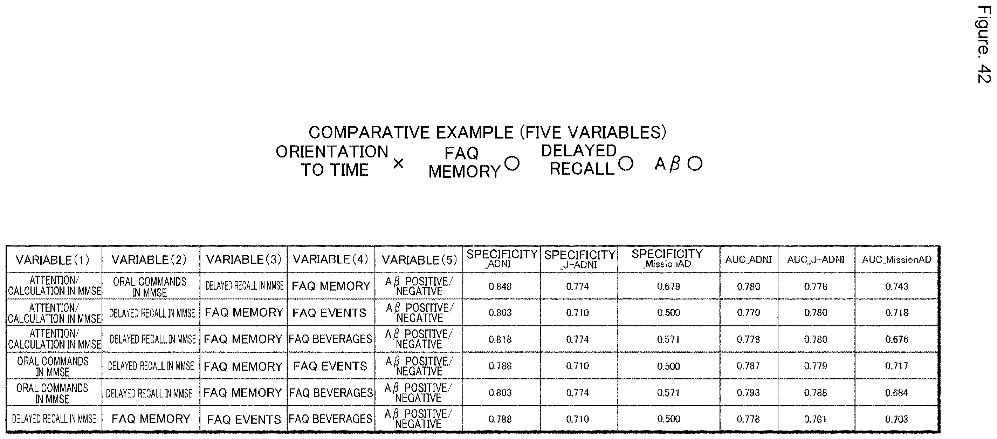

Figure 41 and Figure 42 are diagrams showing an example of training data used for training of the prediction model 132 according to a comparative example. In the example in Figure 41 and Figure 42, training data used for training of the prediction model 132 includes five variables, which are variable (1), variable (2), variable (3), variable (4), and variable (5). Variables (1) to (5) include information that does not fall under any one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (5) do not include information relating to orientation to time that is evaluated by MMSE out of the four essential types of information making up the training data for the prediction model 132 according to Example 2 shown in Figure 31 and Figure 32, and include information relating to delayed recall that is evaluated by MMSE, information relating to memory that is evaluated by FAQ, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

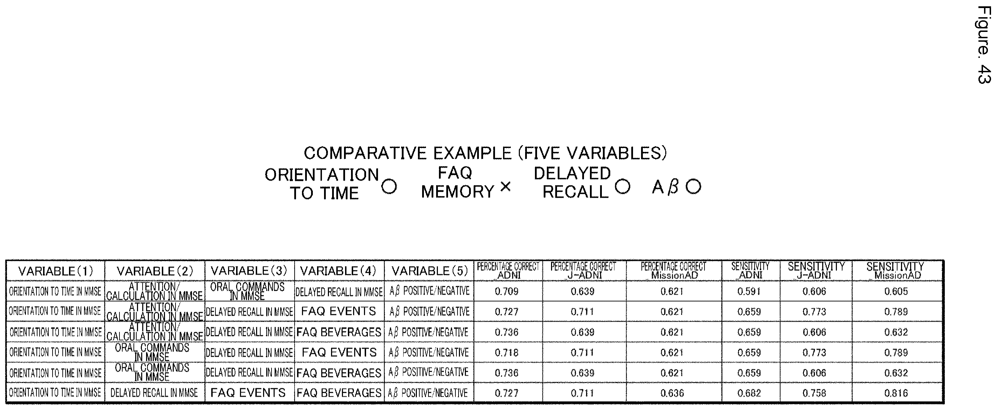

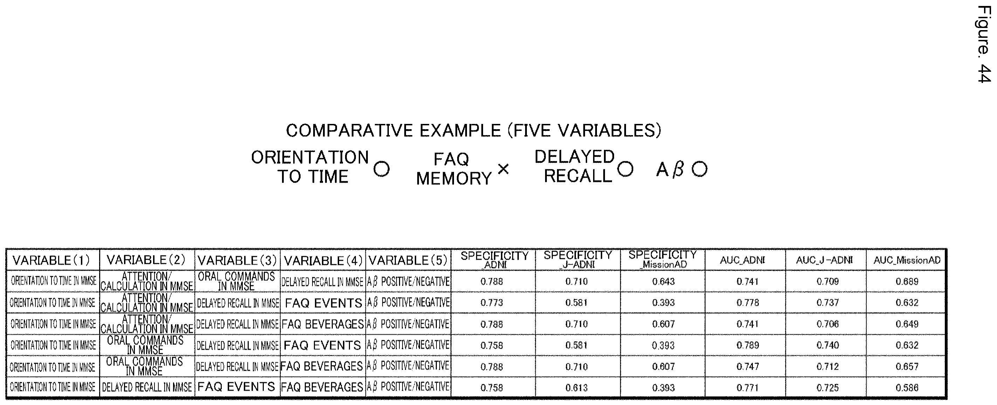

Figure 43 and Figure 44 are diagrams showing an example of training data used for training of the prediction model 132 according to a comparative example. In the example in Figure 43 and Figure 44, training data used for training of the prediction model 132 includes five variables, which are variable (1), variable (2), variable (3), variable (4), and variable (5). Variables (1) to (5) include information that does not fall under any one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (5) do not include information relating to memory that is evaluated by FAQ out of the four essential types of information making up the training data for the prediction model 132 according to Example 2 shown in Figure 31 and Figure 32, and include information relating to orientation to time that is evaluated by MMSE, information relating to delayed recall that is evaluated by MMSE, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

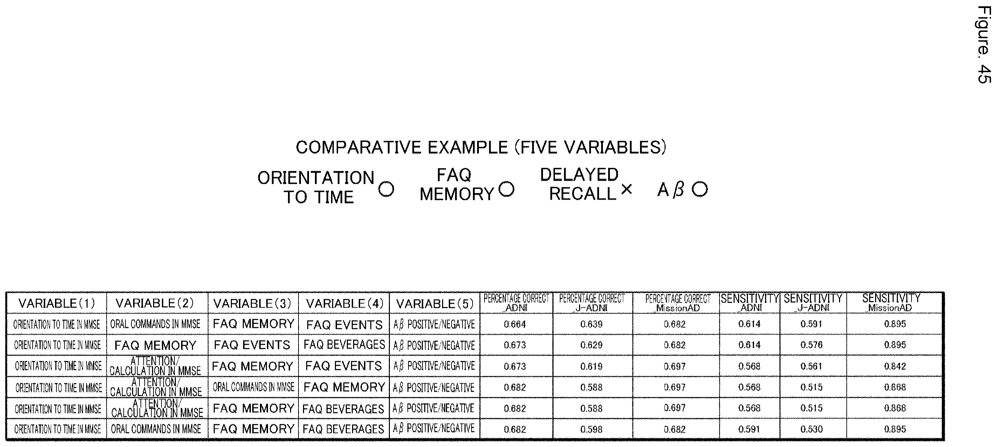

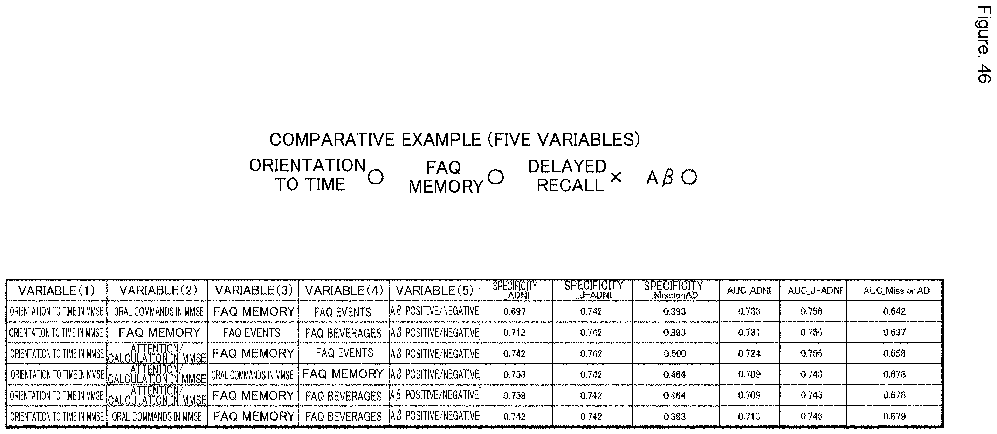

Figure 45 and Figure 46 are diagrams showing an example of training data used for training of the prediction model 132 according to a comparative example. In the example in Figure 45 and Figure 46, training data used for training of the prediction model 132 includes five variables, which are variable (1), variable (2), variable (3), variable (4), and variable (5). Variables (1) to (4) include information that does not fall under any one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (5) do not include information relating to delayed recall that is evaluated by MMSE out of the four essential types of information making up the training data for the prediction model 132 according to Example 2 shown in Figure 31 and Figure 32, and include information relating to orientation to time that is evaluated by MMSE, information relating to memory that is evaluated by FAQ, and information relating to Aβ positive/negative, which are the three remaining types of essential information. In this example, evaluation values of at least one of sensitivity and AUC are lower than "0.7" in each evaluation of the prediction model 132 using ADNI, J-ADNI, and MissionAD.

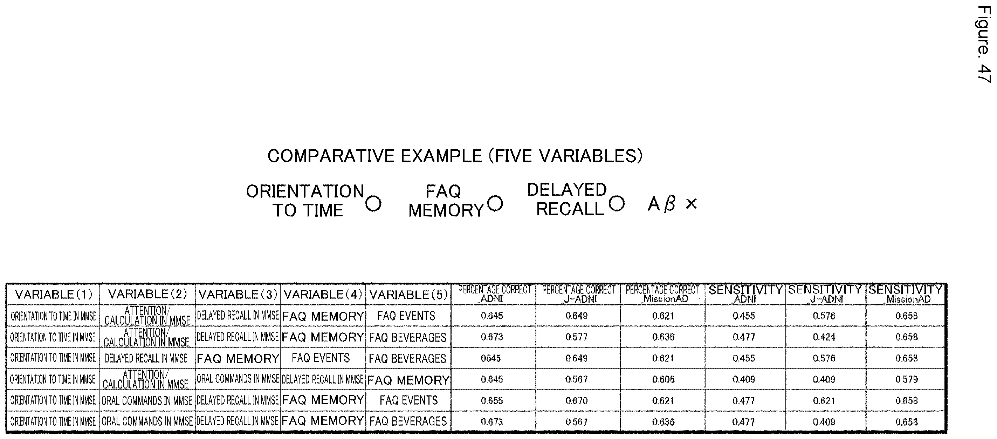

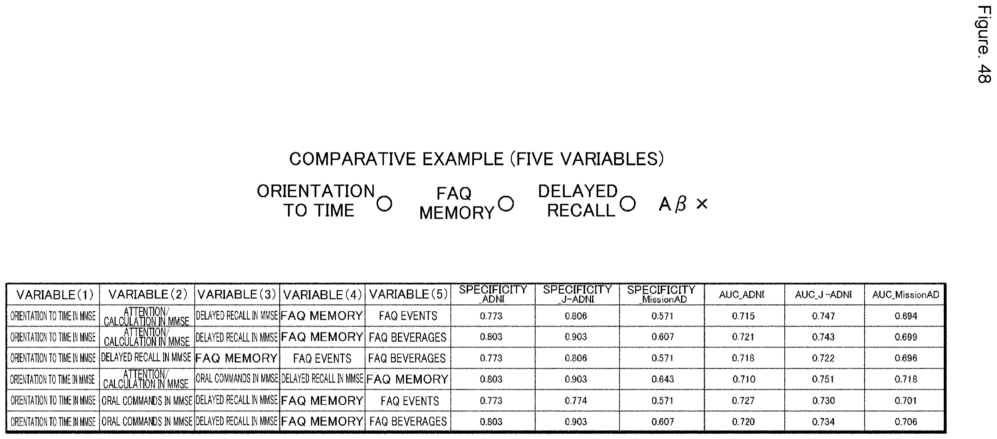

Figure 47 and Figure 48 are diagrams showing an example of training data used for training of the prediction model 132 according to a comparative example. In the example in Figure 47 and Figure 48, training data used for training of the prediction model 132 includes five variables, which are variable (1), variable (2), variable (3), variable (4), and variable (5). Variables (1) to (5) include information that does not fall under any one of first information relating to orientation, second information relating to memory, third information relating to memory of holidays, family gatherings, reservations, or schedule of taking medication in IADL, or memory of meals or actual work of travel in IADL, and fourth information relating to amyloid β positive/negative. Variables (1) to (5) do not include information relating to Aβ positive/negative out of the four essential types of information making up the training data for the prediction model 132 according to Example 2 shown in Figure 31 and Figure 32, and include information relating to orientation to time that is evaluated by MMSE, information relating to delayed recall that is evaluated by MMSE, and information relating to memory that is evaluated by FAQ, which are the three remaining types of essential information. In this example, evaluation values of at least one of sensitivity and AUC are lower than "0.7" in each evaluation of the prediction model 132 using ADNI, J-ADNI, and MissionAD.

According to the information processing device 100 described above, the data that is selected as training data for use in training the prediction model 132 is from a broad area such as ADNI, J-ADNI, and MissionAD, that is highly evaluated on the basis of a plurality of evaluation data 131 that is not readily affected by differences in culture, customs, and preferences, and accordingly high prediction precision can be achieved regardless of race, culture, and so forth. Also, data that can be obtained readily easily from patients by questionnaires is selected as training data to be used for training the prediction model 132, and accordingly, interview (questionnaire) time can be reduced, physical load of examination (no biological samples taken) and number of times of hospital visits can be reduced, and thus the processing load on patients and equipment can be alleviated.

### [Hardware Configuration]

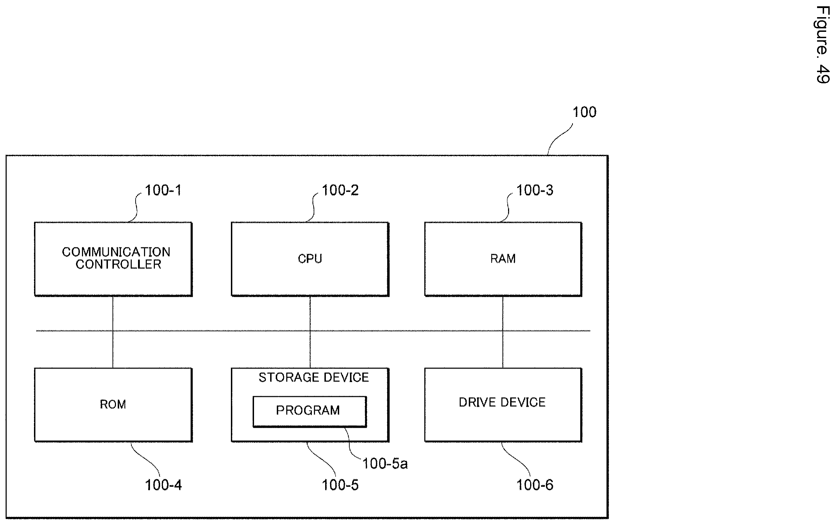

Figure 49 is a diagram illustrating an example of a hardware configuration of the information processing device 100 according to the present embodiment. As can be seen from this drawing, the information processing device 100 is configured with a communication controller 100-1, a CPU 100-2, RAM (Random Access Memory) 100-3 that is used as working memory, ROM (Read Only Memory) 100-4 storing boot programs and so forth, a storage device 100-5 such as flash memory, an HDD (Hard Disk Drive), or the like, a drive device 100-6, and so forth, being connected to each other by an internal bus or a dedicated communication line. The communication controller 100-1 performs communication with components other than the information processing device 100. A program 100-5a that is executed by the CPU 100-2 is stored in the storage device 100-5. This program is loaded to the RAM 100-3 by a DMA (Direct Memory Access) controller (omitted from illustration) or the like, and is executed by the CPU 100-2. Thus, the acquisition unit 121, the prediction unit 122, and the output unit 123 are realized.

Note that the embodiments described above are for facilitating understanding of the present invention, and not for restrictively construing the present invention. The present invention can be changed/modified without departing from the spirit thereof, and equivalents thereof are also included in the present invention. That is to say, arrangements obtained by one skilled in the art modifying design of the embodiments are encompassed by the scope of the present invention as long as they have features of the present invention. Also, the embodiments are exemplary, and it is needless to say that partial substitutions or combinations of configurations shown in different embodiments can be made, and that these are also encompassed by the scope of the present invention as long as they have features of the present invention.

### Reference Signs List

- 10: Terminal device
- 100: Information processing device
- 110: Communication equipment
- 120: Control unit
- 121: Acquisition unit
- 122: Prediction unit
- 123: Output unit
- 130: Storage unit
- 131: Evaluation data
- 132: Prediction model
- NW: Communication network

## Claims

1. An information processing device that
inputs information of a patient to a prediction model trained using training data, in which
at least four variables that fall under at least any of
first information relating to orientation,
second information relating to memory,
third information relating to memory of a holiday, a family gathering, a reservation, or schedule of taking medication in IADL, or memory of a meal or actual work of travel in IADL, and
fourth information relating to positive/negative of a biomarker are associated with
presence/absence of advance of a symptom of mild cognitive impairment (MCI) or mild Alzheimer's dementia (mild AD), with the information, which is input, of the patient corresponding to each of the at least four variables, and that
predicts a probability of advance of a symptom of mild cognitive impairment (MCI) or mild Alzheimer's dementia (mild AD) in the patient, wherein
the at least four variables include
a first variable that is information relating to orientation relating to time out of the first information,
a second variable that is information relating to delayed recall out of the second information,
a third variable that is information relating to memory regarding a holiday, a family gathering, a reservation, or schedule for taking medication in IADL, out of the third information, and
a fourth variable that is information relating to amyloid β positive/negative out of the fourth information.

2. The information processing device according to claim 1, wherein the at least four variables are four variables which are the first variable, the second variable, the third variable, and the fourth variable.

3. The information processing device according to claim 1, wherein the at least four variables are five variables which are the first variable, the second variable, the third variable, the fourth variable, and a fifth variable that is information of any of the first information, the second information, and the third information.

4. The information processing device according to claim 3, wherein the fifth variable is information relating to orientation of a place out of the first information.

5. The information processing device according to claim 3, wherein the fifth variable is information relating to memory of a name or a type of an object out of the second information.

6. The information processing device according to claim 3, wherein the fifth variable is information relating to memory of actual work of meals in IADL out of the third information.

7. The information processing device according to claim 3, wherein the fifth variable is information relating to memory of actual work of travel in IADL out of the third information.

8. An information processing method comprising the steps of:
acquiring patient information;
predicting a probability of advance of a symptom of mild cognitive impairment (MCI) or mild Alzheimer's dementia (mild AD) in a patient, by inputting information to a prediction model trained using training data, in which at least four variables that fall under at least any of first information relating to orientation, second information relating to memory, third information relating to memory of a holiday, a family gathering, a reservation, or schedule of taking medication in IADL, or memory of a meal or actual work of travel in IADL, and fourth information relating to positive/negative of a biomarker are associated with presence/absence of advance of a symptom of mild cognitive impairment (MCI) or mild Alzheimer's dementia (mild AD), with the information that is input corresponding to each of the at least four variables included in the patient information; and
outputting a prediction result relating to the probability of advance of a symptom of mild cognitive impairment (MCI) or mild Alzheimer's dementia (mild AD) in the patient, wherein
the at least four variables include
a first variable that is information relating to orientation relating to time out of the first information,
a second variable that is information relating to delayed recall out of the second information,
a third variable that is information relating to memory regarding a holiday, a family gathering, a reservation, or schedule for taking medication in IADL, out of the third information, and
a fourth variable that is information relating to amyloid β positive/negative out of the fourth information.

9. A program causing a computer to execute:
processing of acquiring patient information;
processing of predicting a probability of advance of a symptom of mild cognitive impairment (MCI) or mild Alzheimer's dementia (mild AD) in a patient, by inputting information to a prediction model trained using training data, in which at least four variables that fall under at least any of first information relating to orientation, second information relating to memory, third information relating to memory of a holiday, a family gathering, a reservation, or schedule of taking medication in IADL, or memory of a meal or actual work of travel in IADL, and fourth information relating to positive/negative of a biomarker are associated with presence/absence of advance of a symptom of mild cognitive impairment (MCI) or mild Alzheimer's dementia (mild AD), with the information that is input corresponding to each of the at least four variables included in the patient information; and
processing of outputting a prediction result relating to the probability of advance of a symptom of mild cognitive impairment (MCI) or mild Alzheimer's dementia (mild AD) in the patient, wherein
the at least four variables include
a first variable that is information relating to orientation relating to time out of the first information,
a second variable that is information relating to delayed recall out of the second information,
a third variable that is information relating to memory regarding a holiday, a family gathering, a reservation, or schedule for taking medication in IADL, out of the third information, and
a fourth variable that is information relating to amyloid β positive/negative out of the fourth information.

10. A computer-readable non-transitory storage medium, storing the program according to claim 9.
